# EUROPÄISCHE PATENTSCHRIFT

(11) **EP 4 070 842 B1**
(45) Veröffentlichungstag und Bekanntmachung des Hinweises auf die Patenterteilung: **16.07.2025**
(21) Anmeldenummer: 22163665.7
(22) Anmeldetag: 22.03.2022
(51) Int. Cl.: A61M 16/06

(54) **VERSTELLBARE STIRNSTÜTZE FÜR EINE MASKE**
ADJUSTABLE FOREHEAD SUPPORT FOR A MASK
SUPPORT FRONTAL RÉGLABLE POUR UN MASQUE

(30) Priorität: 09.04.2021 DE 102021001836
(43) Veröffentlichungstag der Anmeldung: 12.10.2022
(73) Patentinhaber: Löwenstein Medical Technology S.A., 2557 Luxembourg (LU)
(72) Erfinder: Frerichs, Arnold, 21614 Buxtehude (DE); Gardein, Joachim, 38430 Icod de los Vinos (ES); Bechtel, Martin, 21423 Winsen / Luhe (DE)
(74) Vertreter: Löwenstein Medical IP

(56) Entgegenhaltungen:
- EP-A1- 2 329 858
- WO-A1-2016/197195
- WO-A2-2010/133218
- US-A1- 2014 000 617

## Beschreibung

Die Erfindung betrifft eine verstellbare Stirnstütze für eine Maske.

Um Atemgas von einem Beatmungsgerät an einen Patienten abzugeben stellen Masken in der Regel die Schnittstelle zwischen Patient und Beatmungsgerät dar. Neben einem gasdichten Sitz der Maske auf dem Gesicht des Patienten ist eine bequeme und genaue Passform der Maske ein wichtiges Kriterium.

Neben einem Maskenkissen, welches rund um Nase und/oder Mund des Patienten anliegt, spielt auch die Ausrichtung auf dem Gesicht eine Rolle. Eine Möglichkeit den Sitz der Maske zu beeinflussen stellt dabei auch die Stirnstütze dar. Aus dem Stand der Technik sind verschiedene Stirnstützen bekannt, häufig sind diese aber nicht verstellbar, bewegen sich nur entlang einer Richtung oder besitzen einen klobigen und/oder komplizierten Aufbau.

WO 2010/133218 A2 offenbart eine Maske mit einer Stirnstützverstellung zum Verstellen eines Stirnpolsters der Maske gegenüber einem Maskenkörper. Die Stirnstützverstellung umfasst einen feststehenden Stützarm und einen drehbar mit dem feststehenden Stützarm verbundenen beweglichen Stützarm. Das Stirnpolster ist einerseits verschiebbar mit dem feststehenden Stützarm und andererseits drehbar mit dem beweglichen Stützarm verbunden. Der feststehende Stützarm weist einen Schieber auf, der in Längsrichtung des feststehenden Stützarms verschiebbar ist und in Führungsnuten im beweglichen Stützarm eingreift. Durch Verschieben des Schiebers in die eine oder andere Richtung wird das Stirnpolster entweder hin zum oder weg vom feststehenden Stützarm bewegt.

Weitere Beispiele für Masken mit verstellbaren Stirnstützen sind in US 2014/000617 A1, EP 2 329 858 A1 und WO 2016/197195 A1 offenbart.

Aufgabe der vorliegenden Erfindung ist daher die Bereitstellung einer verstellbaren Stirnstütze welche einen sicheren und bequemen Sitz der Maske ermöglicht. Die Aufgabe wird durch eine Stirnstützverstellung für eine Maske gemäß Anspruch 1 gelöst.

### ZUSAMMENFASSUNG DER ERFINDUNG

Die vorliegende Erfindung betrifft eine Stirnstützverstellung für eine Maske gemäß Anspruch 1. Weitere bevorzugte Ausführungsformen der Erfindung sind in den abhängigen Ansprüchen definiert.

### ZUSAMMENFASSUNG DER TECHNOLOGIE

In der folgenden Beschreibung wird auf verschiedene Beispiele oder Aspekte der vorliegenden Technologie verwiesen. Hierin offenbarte Beispiele oder Aspekte, die nicht in den Umfang der beigefügten Ansprüche fallen, bilden nicht Teil der vorliegenden Erfindung, sind aber für das Verständnis der Prinzipien der Erfindung nützlich.

Der Schutzumfang der vorliegenden Erfindung wird durch die beigefügten Ansprüche definiert.

In einem ersten Aspekt betrifft die Technologie eine Stirnstützverstellung für eine Maske, wobei die Stirnstützverstellung zumindest eine Stirnstütze, bestehend aus Stützarm und Polsterhalter, ein Verbindungselement, eine Schiene mit zumindest einer Führungsnut, ein Schiebeelement umfasst und wobei das Schiebeelement zumindest eine Schieberplatte, zumindest einen Zapfen sowie ein Führungselement aufweist, wobei der zumindest eine Zapfen in der zumindest einen Führungsnut der Schiene dreh- und schiebbar gelagert ist und wobei das Schiebeelement mit dem Stützarm beweglich verbunden ist. Die Stirnstützverstellung wird dadurch gekennzeichnet, dass die Stirnstützverstellung so eingerichtet ist, dass ein Verschieben des Schiebeelements entlang der Schiene zu einem Schwenken der Stirnstütze um mindestens eine Schwenkachse D führt.

In manchen Ausführungsformen kennzeichnet die Stirnstützverstellung, dass zumindest eine Innenwand an der Schieberplatte angeordnet ist, wobei an der Innenwand der zumindest eine Zapfen und das zumindest eine Führungselement angeordnet sind.

In manchen Ausführungsformen kennzeichnet die Stirnstützverstellung, dass in der Schiene zumindest zwei Einkerbungen angeordnet sind und an zumindest einem Zapfen eine Rastnase angeordnet ist, welche in den Einkerbungen einrasten und dadurch eine Schwenkstellung fixieren kann.

In manchen Ausführungsformen kennzeichnet die Stirnstützverstellung, dass die Schieberplatte an zwei sich gegenüberliegenden Seiten jeweils einen Flügel aufweist, welche im Wesentlichen senkrecht zur Schieberplatte stehen.

In manchen Ausführungsformen kennzeichnet die Stirnstützverstellung, dass die Flügel zumindest jeweils zwei Unterflügel aufweisen, wobei zwischen den jeweiligen Unterflügeln ein Zwischenraum vorhanden ist.

In manchen Ausführungsformen kennzeichnet die Stirnstützverstellung, dass der Stützarm zumindest einen Träger aufweist, welcher über zumindest einen Pin mit dem Verbindungselement drehbar verbunden ist.

In manchen Ausführungsformen kennzeichnet die Stirnstützverstellung, dass auf zumindest einer Seite des zumindest einen Trägers diskrete Bezeichnungen zur Markierung der Schwenkstellung angeordnet sind, wobei die diskreten Bezeichnungen so angeordnet sind, dass zumindest eine Bezeichnung im Zwischenraum zwischen den Unterflügeln zu sehen ist, wenn die Rastnase die entsprechende Schwenkstellung fixiert.

In manchen Ausführungsformen kennzeichnet die Stirnstützverstellung, dass die Schiene und das Verbindungselement in den Maskenkörper integriert sind.

In manchen Ausführungsformen kennzeichnet die Stirnstützverstellung, dass der Stützarm zumindest zwei Träger aufweist, wobei die Träger parallel zueinander angeordnet sind und über Verbindungen miteinander verbunden sind.

In manchen Ausführungsformen kennzeichnet die Stirnstützverstellung, dass die Verbindungen zusammen mit den Trägern einen Freiraum bilden, durch welchen das Schiebeelement zumindest teilweise gesteckt ist.

In manchen Ausführungsformen kennzeichnet die Stirnstützverstellung, dass an der Schieberplatte zumindest zwei Innenwände angeordnet sind, welche im Wesentlichen senkrecht an der Schieberplatte angeordnet sind und wobei an den Kanten, welche der Schieberplatte gegenüberliegen, Zapfen und die Führungselemente angeordnet sind.

In manchen Ausführungsformen kennzeichnet die Stirnstützverstellung, dass die Träger zwischen der Schieberplatte und den Führungselementen geklemmt sind.

In manchen Ausführungsformen kennzeichnet die Stirnstützverstellung, dass die Träger des Stützarms zumindest eine Führungskante aufweisen, an welcher die Führungselemente entlanggeführt werden.

In manchen Ausführungsformen kennzeichnet die Stirnstützverstellung, dass die Führungselemente und Zapfen in einem Abstand J zueinander angeordnet sind.

In manchen Ausführungsformen kennzeichnet die Stirnstützverstellung, dass die Führungskante und eine Oberkante der Führungsnut einen Abstand K zueinander aufweisen, wenn die Führungskante und die Führungsnut parallel zueinander verlaufen.

In manchen Ausführungsformen kennzeichnet die Stirnstützverstellung, dass der Abstand J nicht gleich dem Abstand K ist.

In manchen Ausführungsformen kennzeichnet die Stirnstützverstellung, dass sich der Abstand J beim Bewegen des Schiebeelements nicht verändert.

In manchen Ausführungsformen kennzeichnet die Stirnstützverstellung, dass die Führungsnut geschwungen oder gerade ausgeführt ist, wobei eine geschwungene Führungsnut einen Radius R1 aufweist.

In manchen Ausführungsformen kennzeichnet die Stirnstützverstellung, dass die Führungskante (30, 49) geschwungen oder gerade ausgeführt ist, wobei eine geschwungene Führungskante (30, 49) einen Radius R2 aufweist.

In manchen Ausführungsformen kennzeichnet die Stirnstützverstellung, dass der Radius R1 nicht gleich dem Radius R2 ist, wobei das Verhältnis R2:R1 oder R1:R2 in einem Bereich von 0,5 bis 0,99, bevorzugt 0,7 bis 0,99, mehr bevorzugt 0,75 bis 0,985 liegt.

In manchen Ausführungsformen kennzeichnet die Stirnstützverstellung, dass die Radien R1 und R2 in einem Bereich zwischen 7 cm und 15 cm liegen, bevorzugt zwischen 9 cm und 11 cm.

In manchen Ausführungsformen kennzeichnet die Stirnstützverstellung, dass die Stirnstütze um einen maximalen Schwenkwinkel C geschwenkt werden kann, wobei der maximale Schwenkwinkel C zwischen 10° und 40° liegt, bevorzugt zwischen 15° und 25°.

In manchen Ausführungsformen kennzeichnet die Stirnstützverstellung, dass die Oberkante der Führungsnut auf einem Kreis K1 mit dem Radius R1 liegt und die Führungskante auf einem Kreis K2 mit dem Radius R2 liegt.

In manchen Ausführungsformen kennzeichnet die Stirnstützverstellung, dass die Schwenkachse D immer die gleiche relative Position, also innerhalb oder außerhalb liegend, zu den Kreisen K1 und K2 hat, wenn der maximale Schwenkwinkel C nicht überschritten wird.

In manchen Ausführungsformen kennzeichnet die Stirnstützverstellung, dass die Schwenkachse D immer außerhalb des Kreises K2 liegt und immer innerhalb der Kreises K1.

In manchen Ausführungsformen kennzeichnet die Stirnstützverstellung, dass ein Schieben des Schiebeelements entlang der Schiene in Richtung Polsterhalter ein Annähern des Stützarms an den Maskenkörper bewirkt.

In manchen Ausführungsformen kennzeichnet die Stirnstützverstellung, dass der Stützarm an einem Ende in den Polsterhalter übergeht wobei an dem Ende des Stützarms, welches dem Ende mit dem Polsterhalter gegenüberliegt, an der Verbindung ein Stift angeordnet ist, über welchen ein Verschluss unverlierbar mit der Maske verbunden ist.

In manchen Ausführungsformen kennzeichnet die Stirnstützverstellung, dass der Polsterhalter einen Halterrahmen aufweist, welcher zusammen mit einer Haltermitte einen Freiraum bildet, in welchen zumindest eine Polsteraufnahme von zumindest einer Seite des Halterrahmens, in Richtung Haltermitte hineinragt und zur Aufnahme eines Stirnpolsters dient.

In manchen Ausführungsformen kennzeichnet die Stirnstützverstellung, dass die Zapfen und Führungselemente so angeordnet und ausgebildet sind, dass ein Schieben des Schiebeelements zu einem Verschieben der Zapfen entlang der Führungsnut und ein Verschieben der Führungselemente entlang der Führungskante erfolgt, wobei gleichzeitig die Stirnstütze um die Schwenkachse D schwenkt und das Schiebeelement um die Zapfen gedreht wird.

In einem weiteren Aspekt betrifft die Technologie eine Maske mit einer solchen Stirnstützverstellung, wobei zwischen Anschluss und Verbindungselement ein Gasanschluss angeordnet ist.

In manchen Ausführungsformen kennzeichnet die Maske, dass der Gasanschluss ein Anschluss für eine zusätzliche Sauerstoffversorgung ist.

In manchen Ausführungsformen kennzeichnet die Maske, dass der Gasanschluss mit einem Verschluss verschließbar ist, wobei dieser Verschluss unverlierbar mit der Maske über einen Stift an der Stirnstütze verbunden ist.

Es ist darauf hinzuweisen, dass eine hierin verwendete, zwischen zwei Merkmalen stehende und diese miteinander verknüpfende Konjunktion "und/oder" stets so auszulegen ist, dass in einer ersten Ausgestaltung des Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Maske kann, soweit nicht ausdrücklich anders beschrieben, jegliches Peripheriegerät verstanden werden, welches zur Interaktion, insbesondere zu Therapie- oder Diagnosezwecken, mit einem Lebewesen gedacht ist. Die Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske eingesetzt werden.

Das Fixieren einer Schwenkstellung und/oder Schwenkposition durch eine oder mehrere Rastnasen bedeutet eine lösbare Fixierung. In der fixierten Stellung ist dabei in der Regel ein Kraftaufwand notwendig um das Schiebeelement aus der fixierten Position zu bewegen und zu verschieben.

Die Technologie wird anhand der Figuren 1 bis 18 beispielhaft näher beschrieben.
Figur 1: Beatmungsmaske in Perspektivansicht
Figur 2: Maskenkörper mit Stirnstütze
Figur 3: Perspektivische Explosionsansicht Maskenkörper mit Stirnstütze
Figur 4: Seitliche Explosionsansicht Maskenkörper mit Stirnstütze
Figur 5: Frontansicht Maskenkörper mit Stirnstützverstellung zur Darstellung der Symmetrieebene
Figur 6: Längsschnitt durch Stirnstütze und Maskenkörper
Figur 7: Frontansicht Maskenkörper
Figur 8: Seitansicht Maskenkörper
Figur 9: Frontansicht Stirnstütze
Figur 10: Längsschnitt durch Stirnstütze
Figur 11: Detailansicht Stützarm im Bereich Pins zur Verbindung mit dem Verbindungselement
Figur 12: Unteransicht Schiebeelement
Figur 13: Rückansicht Schiebeelement
Figur 14: Längsschnitt Schiebeelement
Figur 15: Seitansicht Schiebeelement
Figur 16: Querschnitt durch Stützarm
Figur 17: Querschnitt durch Schiene/Stützarm/Schiebeelement
Figur 18: Schwenkbereich Stirnstütze
Figur 19: schematische Darstellung der Lage der Führungsnut und der Führungskante in Bezug auf die Kreise K1 und K2.
Figur 20: schematische Darstellung der Funktionsweise der Stirnstützverstellung
Figur 21: schematische Darstellung der Abstände zwischen Zapfen und Führungselementen
Figur 22: schematische Darstellung der Abstände zwischen Führungsnut und Führungskante

Die in den Figuren zum Teil dargestellten Koordinatensysteme dienen primär der Verdeutlichung der Ausrichtung der Ansicht, dabei beschreiben die x-, y- und z-Achsen - soweit nicht anders beschrieben - jeweils die gleiche Richtung, die mit x-Achse einer Figur bezeichnete Richtung entspricht also der Richtung der x-Achse einer anderen Figur.

In den Beschreibungen wird davon ausgegangen, dass der Maskenkörper also solcher eine feste Positionierung hat und durch die Stirnstützverstellung die Stirnstütze bewegt wird. An dieser Stelle sei angemerkt, dass auch andersherum die Stirnstütze als feststehend betrachtet werden kann und durch die Stirnstützverstellung der Maskenkörper bewegt wird. Letztlich findet durch die Stirnstützverstellung eine relative Bewegung zwischen Maskenkörper und Stirnstütze statt.

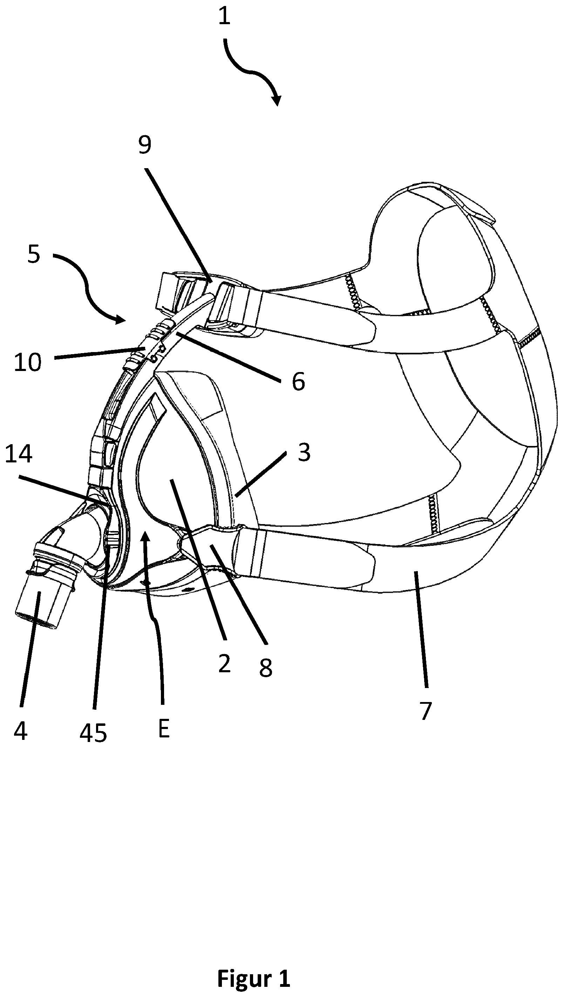

Figur 1 zeigt eine beispielhafte Ausführungsform der Stirnstützverstellung - zumindest bestehend aus Stirnstütze 5, Schiebeelement 10, Schiene 17 (nicht sichtbar in Figur 1) und Verbindungselement 16 (nicht sichtbar in Figur 1) - an einer Maske 1, welche beispielhaft zur Anwendung mit einem Beatmungsgerät ausgebildet ist. Dazu ist mit dem Maskenkörper 2 beispielsweise ein Schlauchanschluss 4 verbunden, an welchen ein Schlauchsystem angeschlossen werden kann, durch welches beispielsweise Atemgas zu und vom Anwender gefördert wird. Der Anschluss 14 ist beispielsweise so eingerichtet, dass verschiedene Arten von Schlauchanschlüssen 4 mit der Maske 1 verbunden werden können. Der Anschluss 14 umfasst in manchen Ausführungsformen zudem Ausatemstrukturen, durch welche Atemgas entweichen kann.

Die Stirnstützverstellung dient der Anpassung der Position der Stirnstütze 5 beziehungsweise des Stirnpolsters 11/der Polsterhalterung 9 relativ zum Maskenkörper 2. Der Erfindung entsprechend besteht die Stirnstützverstellung zumindest aus der Stirnstütze 5, einem Schiebeelement 10, einer Schiene 17, in welchem das Schiebeelement 10 dreh- und schiebbar gelagert ist sowie einem Verbindungselement 16, über welches die Stirnstütze 5 dreh- bzw. schwenkbar mit dem Maskenkörper 2 verbunden ist. Die Anpassung der Position des Stirnpolsters 11/der Polsterhalterung 9 relativ zum Maskenkörper 2 erfolgt durch ein Schwenken der Stirnstütze 5 um eine Schwenkachse D, welche durch die Verbindung der Stirnstütze 5 mit dem Verbindungselement 16 durch Pins 27 definiert wird.

In manchen Ausführungsformen können auch mehrere Schwenkachsen ausgebildet sein. Beispielsweise können die Pins 27 in dem Verbindungselement 16 verschiedene Positionen annehmen, sodass zum einen in den verschiedenen Positionen jeweils eine Schwenkachse zu finden ist. Auch werden die Zapfen 25 des Schiebeelements 10 durch eine Bewegung zwischen den verschiedenen Positionen im Verbindungselement 16 zu einer Schwenkachse.

Der Maskenkörper 2 ist in der Regel schalenförmig mit mindestens zwei Öffnungen ausgebildet, sodass eine Innenseite F und eine Außenseite E des Maskenkörpers 2 erkennbar sind. Die Innenseite F liegt dabei im Innenraum, also in dem schalenförmigen Maskenkörper, welcher im Wesentlichen ein Volumen umschließt. Die Außenseite E des Maskenkörpers 2 liegt dementsprechend außerhalb des Maskenkörpers.

Mit dem Maskenkörper 2 der Maske 1 ist zudem zumindest eine Kopfbänderung 7 verbunden. In der gezeigten Ausführungsform ist die Kopfbänderung 7 beispielsweise einteilig ausgeführt, wobei eine Verbindung mit der Maske 1 im Bereich des Maskenkörpers 2, hier beispielsweise über Bänderclips 8 realisiert, und über den Polsterhalter 9 der Stirnstütze 5 ausgebildet ist. Neben der gezeigten, einteilig ausgeführten Kopfbänderung 7 ist zudem auch eine mehrteilig ausgeführte Kopfbänderung 7 denkbar. So kann ein Teil der Kopfbänderung 7 nur im Bereich des Maskenkörpers 2 mit der Maske 1 verbunden sein und ein weiterer Teil der Kopfbänderung 7 nur über die Stirnstütze 5 (zum Beispiel über den Polsterhalter 9).

Für ein gasdichten Sitz der Maske 1 auf dem Gesicht eines Anwenders ist weiter ein Gesichtspolster 3 mit dem Maskenkörper 2 verbunden. An dieser Stelle ist anzumerken, dass grundsätzlich jegliche Art von Gesichtspolster 3 Verwendung finden kann. Es wird an dieser Stelle davon ausgegangen, dass dem Fachmann bewusst ist, wie der Maskenkörper 2 entsprechend auf verschiedene Gesichtspolsters 3 hinsichtlich einer Verbindung mit dem Maskenkörper 2 anzupassen wäre. In manchen Ausführungsformen ist die Stirnstützverstellung auch dazu ausgebildet in einer Maske 1 angeordnet und verwendet zu werden, welche eine einteilige Ausführung des Maskenkörpers 2 mit Gesichtspolster 3 darstellt. Die Stirnstütze 5 mit Stirnstützverstellung ist beispielsweise außen, d.h. an der Außenseite E, an dem Maskenkörper 2 angeordnet. Die Stirnstütze 5 lässt sich dabei in den Stützarm 6 und den Polsterhalter 9 unterteilen. Der Polsterhalter 9 dient beispielsweise der Aufnahme des Stirnpolsters 11 sowie optional auch der Halterung einer Kopfbänderung 7. Der Stützarm 6 stellt eine Verbindung zwischen Polsterhalter 9 und dem Maskenkörper 2 dar. Durch die Stirnstützverstellung ist das Anpassen der Position der Polsterhalters 9 durch Schwenken der Stirnstütze 5 um eine Schwenkachse D, welche an dem Ende des Stützarms 6 liegt, welches dem Polsterhalter 9 gegenüberliegt, möglich.

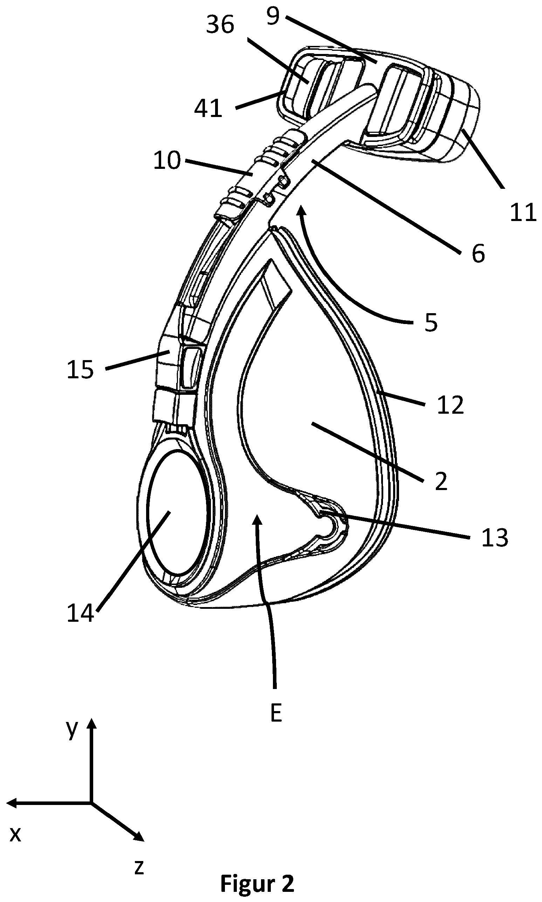

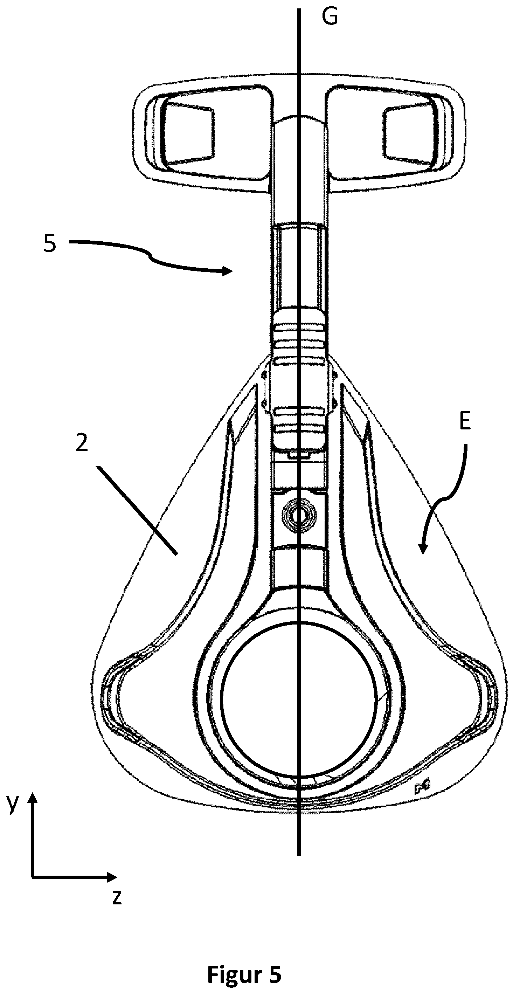

Figur 2 zeigt eine perspektivische Ansicht einer beispielhaften Ausführungsform des Maskenkörpers 2 mit der Stirnstützverstellung. Der Maskenkörper 2 verfügt beispielsweise über einen Polsteranschluss 12 zur Verbindung mit einem Gesichtspolster 3 sowie einem Anschluss 14, beispielsweise zur Verbindung mit einem Schlauchanschluss 4. In manchen Ausführungsformen kann der Anschluss 14 auch so ausgeführt sein, dass beispielsweise ein Filterelement angeschlossen werden kann, beispielsweise auch ohne Schlauchsystem zur Zuführung von Atemgas. Der Teil des Maskenkörpers 2, in welchem der Anschluss 14 angeordnet ist, kann beispielsweise als unterer Teil der Maske angesehen werden. Gewöhnlich ist die Form des Maskenkörpers 2 im Wesentlichen spiegelsymmetrisch ausgebildet, wobei die Spiegelebene G mittig durch den Maskenkörper 2 verläuft (siehe Figur 5). Insbesondere funktionelle und kosmetische Details können dabei durchaus von der Symmetrie abweichen, wie zum Beispiel asymmetrisch angeordnete Führungslinien, beispielsweise für eine Verriegelung eines Schlauchanschlusses 4 in dem Anschluss 14. Der Anschluss 14 ist beispielsweise unten, mittig im Maskenkörper 2 angeordnet, so dass die Symmetrieebene G mittig durch den Anschluss 14 verläuft.

Seitlich an der Außenseite E des Maskenkörpers sind beispielsweise Cliphalter 13 angeordnet, welche Bänderclips 8 mit der Kopfbänderung 7 aufnehmen können. Beispielsweise werden die Bänderclips 8 in den Cliphalter 13 eingeclipst, wobei die Bänderclips 8 in der Cliphalterung 13 in zumindest einer Ebene rotieren können und gegebenenfalls auch einen gewissen Grad an Bewegungsfreiheit in andere Richtungen besitzen. Alternativ (oder auch ergänzend zu) einer Kombination aus Cliphalter 13 und Bänderclips 8 sind auch andere Halterungen der Kopfbänderung 7 an dem Maskenkörper 2 möglich.

Oberhalb des Anschlusses 14 ist beispielhaft ein Verschluss 15 angeordnet, welcher einen zusätzlichen, optionalen Gasanschluss 18 verschließt. Der Verschluss 15 ist beispielsweise unverlierbar mit dem Stützarm 6 verbunden. Der Verschluss 15 dient dem Verschließen des Gasanschlusses 18, wenn dieser nicht benötigt wird. In manchen Ausführungsformen ist im Maskenkörper 2 dieser Gasanschluss 18 nicht angeordnet, sodass auch der Verschluss 15 nicht angeordnet ist.

Die Stirnstütze 5 ist, als Teil der Stirnstützverstellung, oberhalb des Verschlusses 15 beziehungsweise des Gasanschlusses 18 an der Außenseite E des Maskenkörpers 2 angeordnet aber nicht Teil des Maskenkörpers 2. An einem Ende des Stützarms 6 ist die Stirnstütze 5 schwenkbar mit dem Maskenkörper 2 über zumindest ein Verbindungselement 16 des Maskenkörpers 2 verbunden. An dem anderen Ende des Stützarms 6 ist der Polsterhalter 9 angeordnet, welcher zum einen zur Aufnahme zumindest eines Stirnpolsters 11 sowie einer Kopfbänderung 7 ausgebildet ist. Die Kopfbänderung 7 wird beispielsweise durch zumindest einen Spalt 36 der Polsteraufnahme 9 um eine Bänderaufnahme 41 herumgeführt und fixiert. Beispielsweise kann eine Fixierung der Kopfbänderung 7 über eine Art Klettverschluss erfolgen.

Die Stirnstützverstellung umfasst zudem das Schiebeelement 10, welches über Zapfen 25 dreh- und schiebbar in einer Schiene 17 mit dem Maskenkörper 2 als auch schiebbar mit dem Stützarm 6 verbunden ist. Durch Schieben des Schiebelements 10 wird die Stirnstütze 5 um die Schwenkachse D, welche in den mit dem Verbindungselement 26 verbundenen Pins 27 liegt, geschwenkt.

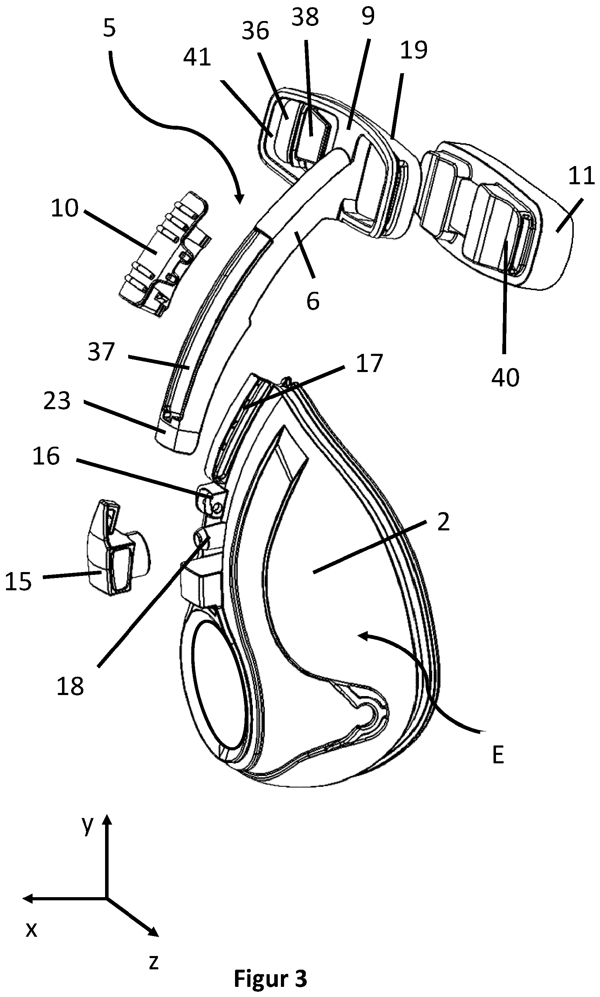

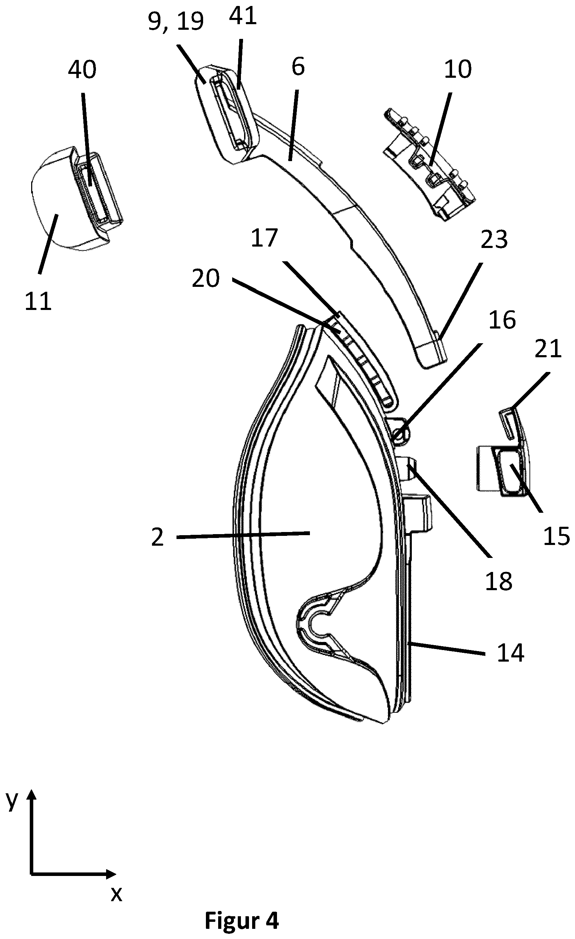

Eine beispielhafte Ausführungsform der Stirnstützverstellung ist mit dem Maskenkörper 2 in einer Explosionsansicht in den Figuren 3 und 4 dargestellt. Figur 3 zeigt dabei eine perspektivische Ansicht, Figur 4 eine Seitansicht.

An dem Maskenkörper 2 sind an der Außenseite E unter anderem die Schiene 17 zur dreh- und schiebbaren Verbindung mit dem Schiebeelement 10, ein Verbindungselement 16 zur beweglichen, drehbaren Verbindung mit dem Stützarm 6 der Stirnstütze 5 und der Gasanschluss 18, welcher von dem Verschluss 15 verschlossen werden kann, ausgebildet und/oder angeordnet. Die Schiene 17 und/oder das Verbindungselement 16 können beispielsweise auf den Maskenkörper 2 aufgesetzt sein oder Teil des Maskenkörpers 2 sein. Sind Schiene 17 und/oder Verbindungselement 16 Teil des Maskenkörpers 2, sind diese materialmäßig mit dem Maskenkörper 2 verbunden und bevorzugt einteilig hergestellt. Bei aufgesetzter Schiene 17 und/oder Verbindungselements 16 werden diese zum Beispiel durch aufstecken, kleben und/oder schrauben mit dem Maskenkörper verbunden.

An einem Ende des Stützarms 6 der Stirnstütze 5 ist die Polsterhalterung 9 angeordnet. Die Polsterhalterung 9 weist einen Halterrahmen 19 auf, von welchem die Bänderhalterung 41 abgeht. Zwischen der Bänderhalterung 41 und dem Halterrahmen 19 ist ein Spalt 36, durch welchen die Kopfbänderung 7 geführt werden kann. An dem Polsterrahmen 19 sind zudem beispielsweise zwei Polsteraufnahmen 38 ausgebildet, über welche die Taschen 40 des Stirnpolsters 11 gesteckt werden können um das Stirnpolster 11 mit dem Polsterhalter 9 zu verbinden. Alternativ können die Taschen 40 auch als Lasche oder Laschen ausgebildet sein, wobei dann die Laschen jeweils über eine der Polsteraufnahmen 38 gezogen werden.

Der Stützarm 6 wird an dem Ende, welches dem Polsterhalter 9 gegenüberliegt, schwenkbar mit dem Verbindungselement 16 verbunden. Die Verbindung mit dem Verbindungselement 6 stellt gleichzeitig auch die Schwenkachse D, um welche der Stützarm 6 beziehungsweise die Stirnstütze 5 schwenkbar ist dar. Der Stützarm 6 weist weiter einen Freiraum 37 auf, durch welchen das Schiebeelement 10 zumindest teilweise gesteckt wird. Die Ausdehnung des Freiraums 37 bestimmt die Strecke, über welche sich das Schiebeelement 10 schieben lässt. Das Schiebeelement 10 wird neben dem Stützarm 6 auch mit der Schiene 17 verbunden. Innerhalb der Schiene 17 ist das Schiebeelement 10 dreh- und schiebbar gelagert, sodass durch das Schieben des Schiebeelements 10 eine Schwenkbewegung der Stirnstütze 5 ermöglicht wird.

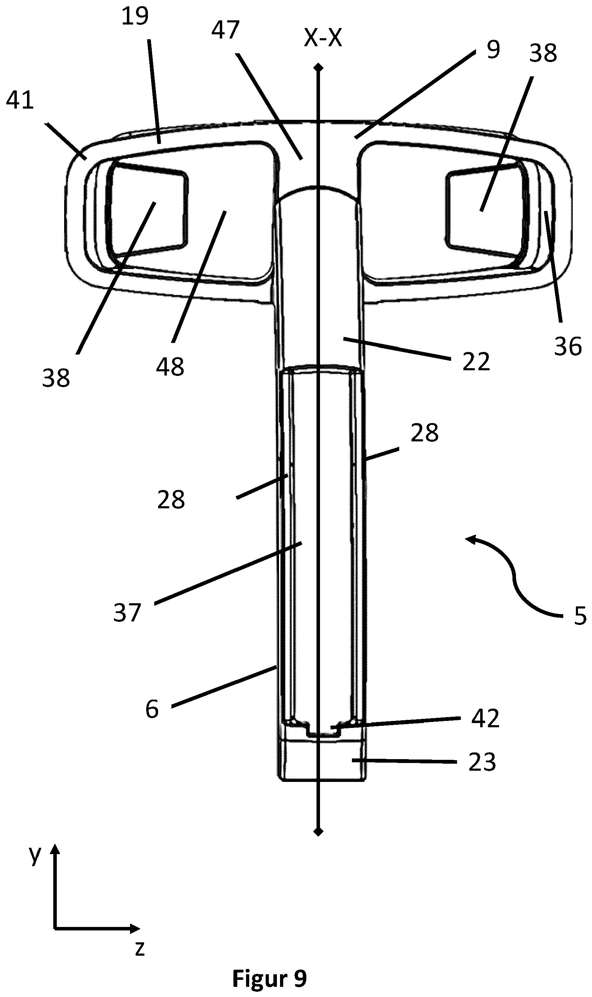

An dem Verschluss 15 ist beispielsweise eine Verschlusslasche 21 angeordnet, mit welcher der Verschluss 15 unverlierbar mit der Maske 1 beziehungsweise der Stirnstütze 5 verbunden werden kann. Dazu weißt die Verschlusslasche 21 beispielsweise ein Loch auf, durch welches ein Stift 39 des Stützarms 6 gesteckt werden kann. Dieser Stift 39 findet beispielsweise Platz in einem Zwischenraum 35 des Verbindungselements 16. Die Verschlusslasche 21 ist beispielsweise weiter so ausgebildet, dass sie um die Verbindung 23 des Stützarms 6 (siehe zum Beispiel Figur 9) gelegt werden kann.

Figur 5 zeigt die Frontansicht (entlang der x-Achse, Figur 4) einer beispielhaften Ausführungsform des Maskenkörpers 2 mit Stirnstützverstellung und Stirnstütze 5. Es ist zu erkennen, dass der Maskenkörper 2 sowie auch die Stirnstütze 5 im Wesentlichen spiegelsymmetrisch sind. Die Symmetrieebene G verläuft dabei mittig durch den Maskenkörper 2 und die Stirnstütze 5 und parallel zu der durch die x- und y-Achse aufgespannten Ebene (siehe Figur 4). Es ist darauf hinzuweisen, dass in der Regel keine perfekte Symmetrie vorliegt und sich die jeweiligen Spiegelbilder in mehreren Details unterscheiden können, als Beispiel sei hier die Ausführung der Schiene 17 des Maskenkörpers 2 genannt, welche in einigen Ausführungsformen einseitig Einkerbungen 33 in der Führungsnut 20 aufweist. Weiter kann zum Beispiel auch der Anschluss 14 so ausgeführt sein, dass asymmetrische Elemente ausgebildet sind oder verschiedene Elemente asymmetrisch angeordnet sind, beispielsweise bei der Führung der Verschlussverriegelung. Die genannten Elemente, welche eine Asymmetrie aufweisen können, dienen an dieser Stelle lediglich einer beispielhaften Erläuterung und stellen keineswegs eine vollständige Aufzählung möglicher Asymmetrien dar.

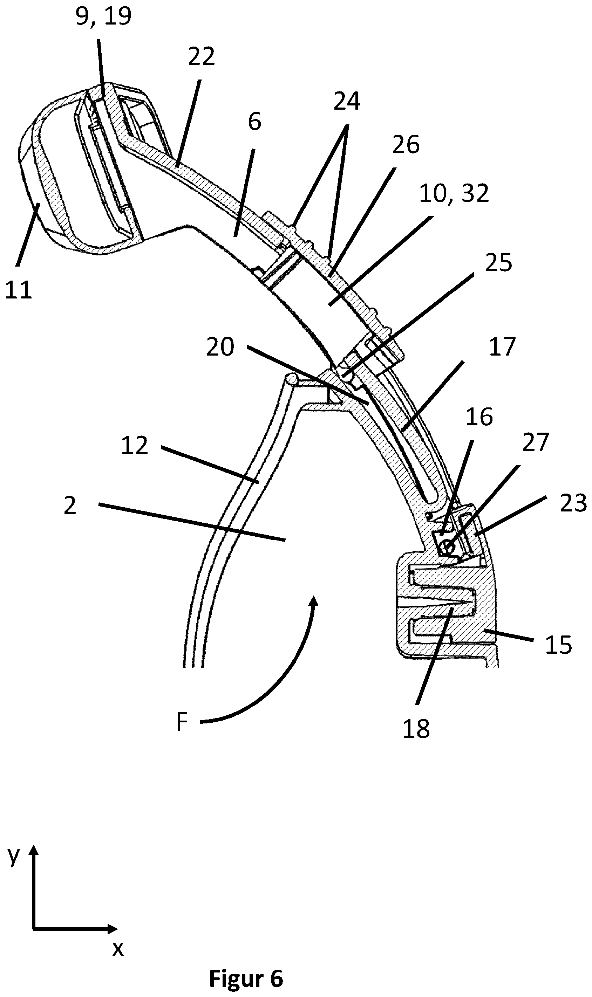

In Figur 6 ist ein Längsschnitt durch eine beispielhafte Ausführungsform des Maskenkörpers 2 und der Stirnstützverstellung, unter anderem umfassend die Stirnstütze 5 und das Schiebeelement 10, dargestellt. Die Schnittebene entsprich dabei im Wesentlichen der Symmetrieebene G, wobei die Schnittebene geringfügig seitlich (in z-Richtung, Figur 5) verschoben ist, sodass nicht genau mittig geschnitten wird.

Der Gasanschluss 18, welcher eine Verbindung des von der Innenseite F umschlossenen Inneren des Maskenkörpers 2 zum Äußeren des Maskenkörpers 2 herstellt, wird in der in Figur 6 gezeigten Darstellung von dem Verschluss 15 verschlossen. Der Verschluss 15 ist unverlierbar mit dem Stützarm 6 verbunden, wobei die Lasche 21 um die Verbindung 23 an einem Ende des Stützarms 6 geführt ist und über den Stift 39 (in Figur 6 nicht dargestellt), welcher an der Verbindung 23 angeordnet ist, gezogen. Der Gasanschluss 18 dient zum Beispiel zum zusätzlichen Einleiten von Sauerstoff oder anderen Gasen in den Innenraum der Maske. Der Gasanschluss 18 ist beispielsweise zwischen Anschluss 14 und Verbindungselement 16 angeordnet, wodurch beim Tragen der Maske 1 durch einen Anwender der Gasanschluss im Bereich der Nase verortet ist.

Die Verbindung 23 verläuft beispielsweise über dem Verbindungselement 16, mit welchem die Stirnstütze 5 über Pins 27 dreh- bzw. schwenkbar verbunden ist. Die Pins 27 stellen so gleichzeitig die Schwenkachse D dar, um welche die Stirnstütze 5 geschwenkt wird.

Oberhalb des Verbindungselements 16 (y-Richtung) ist die Schiene 17 des Maskenkörpers 2 angeordnet. Die Schiene 17 verfügt über zumindest eine Führungsnut 20, in welcher die Zapfen 25 des Schiebeelements 10 schieb- und drehbar gelagert sind. In manchen Ausführungsformen verfügt die Schiene 17 mittig in z-Richtung eine Wand 46, sodass auf beiden Seiten der Wand 46 eine Führungsnut 20 ausgebildet ist. Die Schiene 17, zumindest aber die Führungsnut 20, ist beispielhaft geschwungen geformt und weist einen Radius R1 auf. In manchen Ausführungsformen folgt dieser Radius R1 im Wesentlichen der Form des Maskenkörpers 2. In manchen Ausführungsformen ist der Radius R1 unabhängig von der Form des Maskenkörpers 2. In manchen Ausführungsformen ist die Führungsnut 20 gerade und/oder folgt einer Parabel und/oder einer Freiform. In manchen Ausführungsformen kann die Führungsnut 20 streckenweise zwischen parabelförmig, kreisförmig geschwungen und/oder gerade wechseln.

In manchen Ausführungsformen ist die Führungsnut 20 so ausgeführt, dass die Schwenkachse D auf dem Bogen bzw. Kreis K1 mit Radius R1 liegt, welcher durch die geschwungene Form der Führungsnut 20 beschrieben wird. In manchen Ausführungsformen liegt die Schwenkachse D auch innerhalb des Kreises K1 mit Radius R1, auf dessen Umfang die Führungsnut 20 verläuft. Der Radius R1 beträgt beispielsweise zwischen 7 und 15 cm, bevorzugt zwischen 9 und 11 cm.

Das Schiebeelement 10 ist durch die Zapfen 25, welche dreh- und schiebbar in der Führungsnut 20 gelagert sind, mit dem Maskenkörper 2 beweglich verbunden. Das Schiebeelement 10 lässt sich so in der Schiene 17 schieben, wobei gleichzeitig eine Drehbewegung möglich ist, beispielsweise im Zusammenspiel mit der Stirnstütze 5. Die Zapfen 25 sind beispielsweise an Innenwänden 32 an einer Ecke ausgebildet. Je Innenwand 32 ist jeweils nur ein Zapfen 25 ausgebildet, damit eine Drehbarkeit in der Schiene 17 möglich ist. Die Zapfen 25 liegen dafür auf einer gemeinsamen Achse. Die Innenwände 32 gehen beispielsweise von einer Schieberplatte 26 ab, welche beispielsweise auf dem Stützarm 6 der Stirnstütze 5 aufliegt. Für eine bessere Griffigkeit des Schiebeelements 10 sind beispielsweise unter anderem auf der Schieberplatte Griffstrukturen 24 ausgebildet. Diese Griffstrukturen 24 sind beispielsweise als schwellenartige Erhebungen auf der Schieberplatte 26 ausgebildet. Neben dieser Ausführung sind auch weitere Formen der Griffstrukturen 24, beispielsweise als Noppen oder in Form einer Griffmulde, möglich.

In manchen Ausführungsformen weist das Schiebeelement 10 lediglich eine Innenwand auf, an welcher zum Beispiel beidseitig jeweils ein Zapfen 25 aufweist und auf zumindest einer Seite Führungselemente 29.

Das Schiebeelement 10 stellt ein Bindeglied zwischen Stirnstütze 5 und Maskenkörper 2 dar. Die Stirnstütze 5 ist also sowohl über die Pins 27 in dem Verbindungselement 16, als auch über das Schiebeelement 10 mit dem Maskenkörper 2 verbunden. Dadurch, dass das Schiebeelement 10 in der Schiene 17 drehbar gelagert ist, die Schieberplatte 26 des Schiebeelements 10 aber auf dem Stützarm 6 der Stirnstütze 5 aufliegt, ist ein Schwenken der Stirnstütze 5 nur dann möglich, wenn das Schiebeelement 10 in der Schiene 17 bewegt wird. Ein freies, also unabhängig von dem Schiebeelement 10, Schwenken der Stirnstütze 5 wird durch das Schiebeelement 10 verhindert.

An dem Ende des Stützarms 6, welches nicht über die Pins 27 mit dem Verbindungselement 16 verbunden ist, geht der Stützarm 6 in den Polsterhalter 9 über. Durch den Verbindung 22 wird dabei die Strecke, über welche das Schiebeelement 10 geschoben werden kann begrenzt. Dadurch wird auch verhindert, dass das Schiebeelement 10 aus der Schiene 17 geschoben wird und die Stirnstütze 5 um die Schwenkachse D frei drehbar wird.

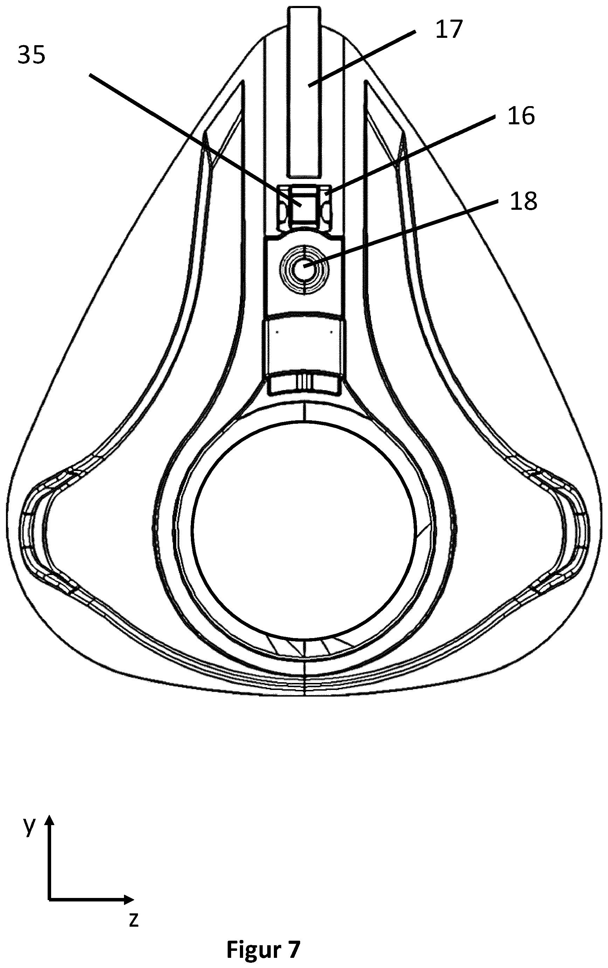

In Figur 7 ist eine beispielhafte Ausführungsform des Maskenkörpers 2 mit der Schiene 17 und dem Verbindungselement 16 in Frontansicht zu sehen. Die Schiene 17 ist im oberen Teil des Maskenkörpers 2 (entlang der y-Achse oberhalb des Gasanschlusses 18) angeordnet. Unterhalb der Schiene 17 ist das Verbindungselement 16 angeordnet. Das Verbindungselement 16 ist beispielhaft aus zwei Wänden aufgebaut, wobei diese Wände jeweils eine Öffnung haben, in welche die Pins 27 eingesteckt werden. In manchen Ausführungsformen können auch die Pins 27 an dem Verbindungselement 16 am Maskenkörper 2 angeordnet sein und der Stützarm 6 weist die entsprechenden Öffnungen auf, in welche die Pins eingesteckt werden. Darüber hinaus sind auch jegliche drehbaren Verbindungsarten zwischen Stützarm 6 und Maskenkörper 2 denkbar. So können beispielsweise sowohl Stützarm 6 als auch Verbindungselement 16 Öffnungen aufweisen, durch welche ein Stift gesteckt wird, welcher die Stirnstütze 5 in dem Verbindungselement 16 drehbar fixiert.

Zwischen den Wänden des Verbindungselements 16 befindet ist beispielsweise ein Zwischenraum 35 ausgebildet, welcher Platz bietet um den Stift 39 aufzunehmen.

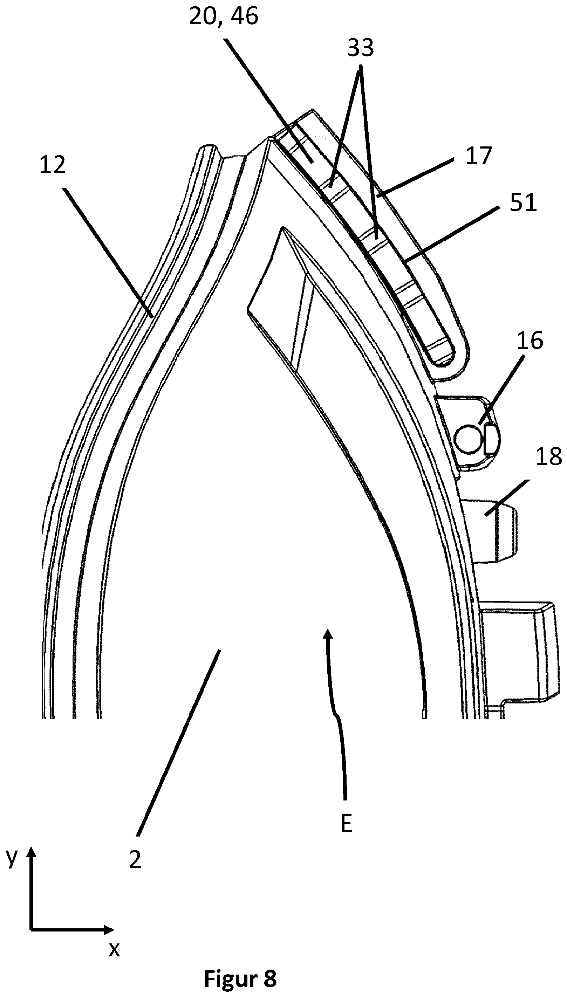

Eine Seitansicht einer beispielhaften Ausführungsform des Maskenkörpers 2 ist in Figur 8 dargestellt. Die Schiene 17 ist zumindest zum Teil in den Maskenkörper 2 integriert. Beispielsweise verfügt die Schiene 17 über eine Wand 46 und weist entsprechend zwei Führungsnuten 20 auf jeder Seite der Wand 46 auf. In der Wand 46 sind zumindest auf einer Seite mehrere, zumindest aber zwei, Einkerbungen 33 ausgebildet. Alternativ oder ergänzend können die Einkerbungen 33 auch auf beiden Seiten der Wand 46 angeordnet sein. Auch eine alternative oder ergänzende Anordnung der Einkerbungen 33 in der Oberkante 51 der Schiene 17 oder der der Oberkante 51 gegenüberliegenden Seite der Schiene 51 ist in einigen Ausführungsformen möglich. Entlang der Führungsnut 20 wird das Schiebeelement 10 mit den Zapfen 25 geführt. In den Einkerbungen 33 der Führungsnut 20 kann eine Rastnase 34, welche an zumindest einem der Zapfen 25 ausgebildet ist, einrasten und damit die eingestellte Position der Stirnstütze 5 feststellen. Die Rastnase 34 ist dabei so an dem Zapfen 25 angeordnet, dass ein einrasten in die Einkerbungen 33 möglich ist. Sind die Einkerbungen 33 beispielsweise in der Oberkante 51 der Schiene 17 angeordnet, so ist die Rastnase 34 beispielsweise an der entsprechenden Seite des Zapfens 25 ausgebildet. In manchen Ausführungsformen weist die Führungsnut 20 keine Einkerbungen auf und auch die Zapfen 25 sind ohne Rastnase 34 ausgeführt. In manchen Ausführungsformen werden auch an anderen Stellen Rastpunkte realisiert oder gar nicht ausgebildet, sodass das Schiebeelement 10 nicht einrasten kann. Die Schiene 17 beziehungsweise zumindest die Führungsnut 20 ist geschwungen ausgebildet und weist einen Radius R1 auf. In alternativen oder ergänzenden Ausführungsformen weist die Führungsnut 20 einen linearen, parabelförmigen und/oder eine Mischform zwischen geschwungen, gerade und/oder parabelförmig auf.

Eine beispielhafte Ausführungsform der Stirnstütze 5 ist in Figur 9 in einer Frontansicht dargestellt. Im groben lässt sich die Stirnstütze 5 in den Stützarm 6 und den Polsterhalter 9 unterteilen, wobei der Stützarm 6 an einem Ende in den Polsterhalter 9 übergeht. Der Poslterhalter 9 beginnt beispielsweise an der Stelle, an welcher der im Wesentlichen geschwungen geformte Stützarm 6 an einer Kante endet. Der Stützarm 6 kann neben einer geschwungenen Form auch gerade und/oder parabelförmig ausgebildet sein. Von dieser Kante breitet sich unter anderem zu den Seiten der Polsterhalter 9 aus. Der Polsterhalter 9 ist gegenüber dem Stützarm 6 um 90° gedreht, wodurch in Frontansicht auf die Stirnstütze 5 im Wesentlichen eine T-Form erkennbar ist. Beispielsweise weist der Polsterhalter 9 somit eine Breite auf, welche gegenüber den anderen Dimensionen mindestens doppelt so groß ist. In manchen Ausführungsformen der Stirnstütze 5 weist der Polsterhalter 9 andere Formen auf. In der in den Figuren (zumindest Figuren 1-5, 8, 9, 18) gezeigten Ausführungsform beschreibt der Polsterhalter 9 in Frontansicht ein Rechteck, kann aber auch alle möglichen anderen Formen annehmen, beispielsweise quadratisch, rund, polygon oder eine Freiform.

Der Polsterhalter 9 dient zu einen der Aufnahme des Stirnpolsters 11 und zum anderen auch zur Aufnahme der Kopfbänderung 7. Das Stirnpolster 11 wird beispielsweise über die Polsteraufnahmen 38 mit dem Polsterhalter 9 verbunden. In der beispielhaften Ausführungsform der Stirnstütze 5 erstrecken sich die Polsteraufnahmen 38 vom Halterrahmen 19 parallel zur eingezeichneten z-Achse nach innen in den vom Halterrahmen 19 und der Haltermitte 47 gebildeten Freiraum 48. Die Polsteraufnahmen 38 überspannen dabei allerdings nicht den gesamten Freiraum von Halterrahmen 19 zu Haltermitte 47, sondern füllen diesen Freiraum 48 nur teilweise. Die Polsteraufnahmen 38 sind beispielsweise als Platten ausgeführt, welcher an einer Kante mit dem Halterrahmen 19 verbunden sind beziehungsweise in diesen übergehen. Über diese Platten können die Taschen 40 des Stirnpolsters 11 gezogen werden, wodurch eine Fixierung des Stirnpolsters 11 auf dem Polsterhalter 9 erfolgt. Die Polsteraufnahmen 38 können in manchen Ausführungsformen auch andere Formen annehmen. Beispielsweise ist es denkbar, dass die Polsteraufnahmen 38 statt als Platten als eine Mehrzahl von Stangen ausgebildet sind, wobei das Stirnpolster 11 dazu statt der Taschen 40 beispielsweise mehrere Ösen oder Laschen aufweist, welche über die Stangen gezogen werden können. In manchen Ausführungsformen des Polsterhalters 9 sind zusätzlich zu den Polsteraufnahmen 38, welche vom Halterrahmen 19 nach innen in Richtung Haltermitte 47 ragen auch noch weitere Polsteraufnahmen angeordnet, welche von der Haltermitte 47 in den Freiraum 48 ragen. Beispielsweise verfügt das dazu passende Stirnpolster eine entsprechende Anzahl an Taschen, welche über die Polsteraufnahmen gezogen werden um das Stirnpolster auf dem Polsterhalter 9 zu fixieren.

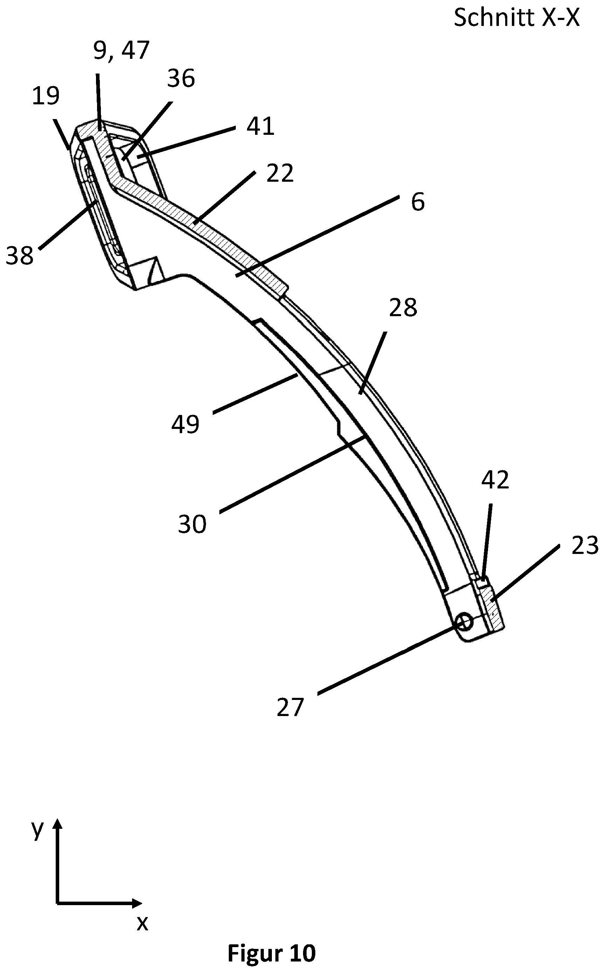

Die Bänderaufnahme 41 bildet mit Halterrahmen 19 einen Spalt 36, durch welchen die Kopfbänderung 7 geführt werden kann und um die Bänderaufnahme 41 gelegt wird. Aus dem Halterahmen 19 geht an den äußeren Seiten (in z-Richtung) jeweils eine Bänderaufnahme 41 hervor. Zwischen der Bänderaufnahme 41 und dem Halterrahmen 19 bildet sich dabei ein Spalt 36, durch welchen die Kopfbänderung 7 durchgeführt und dann um die Bänderaufnahme 41 geschlungen wird. In der in Figuren 9 und 10 gezeigten Ausführungsform der Stirnstütze 5 entsteht der Spalt 36 maßgeblich dadurch, dass die Bänderaufnahme 41 insbesondere in x-Richtung (Koordinatensystem der Figur 10) vom Halterrahmen 19 absteht. In manchen Ausführungsformen kann die Bänderaufnahme 41 auch hauptsächlich in z-Richtung von dem Halterrahmen 19 abgehen.

Der Stützarm 6 der Stirnstütze 5 weist beispielswese zwei Träger 28 auf, welche im oberen und unteren Bereich (gemäß der y-Achse) durch die Verbindung 23 beziehungsweise die Verbindung 22 verbunden sind. Die Träger 28 verlaufen beispielhaft parallel zu einander. Durch die Träger 28 sowie die Kanten der Verbindungen 22 und 23 wird ein Freiraum 37 ausgebildet, durch welchen das Schiebeelement 10 auf den Stützarm 6 aufgesteckt wird. Die Verbindung 22 limitiert dabei, wie weit das Schiebeelement 10 geschoben werden kann und dient in manchen Fällen auch dazu ein Herausschieben des Schiebeelements 10 aus der Schiene 17 zu verhindern.

Die Verbindung 23 am unteren Ende des Stützarms 6 weist beispielsweise eine Lücke 42 auf. Durch diese Lücke 42 kann beispielsweise die Verschlusslasche 21 geführt werden, wenn diese um die Verbindung 23 gelegt wird und über den Stift 39 gezogen wird.

Ein Längsschnitt durch die Stirnstütze 5 entlang der Schnittkante X-X ist in Figur 10 schematisch dargestellt. An einem der Enden des Stützarms 6 sind die Pins 27 angeordnet, welche in das Verbindungselement 16 eingesteckt werden um eine drehbare Verbindung zwischen Stirnstütze 5 und Maskenkörper 2 herzustellen. Die Pins 27 stellen gleichzeitig auch die Schwenkachse D dar, um welche die Stirnstütze 5 geschwenkt werden beziehungsweise rotieren kann. An jeweils einer Kante 49 der zwei Träger 28, welche an einem Ende durch die Verbindung 23 und am anderen Ende durch die Verbindung 22 materialmäßig verbunden sind, ist eine Führungskante 30 ausgebildet, welche auf den Führungselementen 29 des Schiebeelements 10 aufliegen und/oder von dem Schiebelement 10 zwischen Schieberplatte 26 und Führungselemente 29 eingeklemmt werden.

Die Führungskanten 30 sind beispielhaft geschwungen ausgeführt und weisen einen Radius R2 auf, welcher beispielsweise gleich dem Radius R1 der Führungsnut 20 ist. Beispielsweise verläuft der Umfang des Kreises K2 mit Radius R2 auf welchem die Führungskanten verlaufen nicht durch die Schwenkachse D. Beispielsweise liegt die Schwenkachse D außerhalb des Kreises K2 mit Radius R2, auf dessen Umfang die Führungskanten 30 verlaufen. In manchen Ausführungsformen liegt die Schwenkachse D auch auf dem Umfang des Kreises K2 mit Radius R2.

Der Kreis K2 liegt in jedem Fall nicht deckungsgleich mit dem Kreis K1, beide Kreise K1 und K2 haben keinen gemeinsamen Mittelpunkt. Bevorzugt liegen die Kreise K1 und K2 so, dass sie sich gegenseitig schneiden. Die Schnittpunkte von K1 und K2 können dabei in einer Verlängerung der Führungsnut 20 beziehungsweise der Führungskanten 30 liegen.

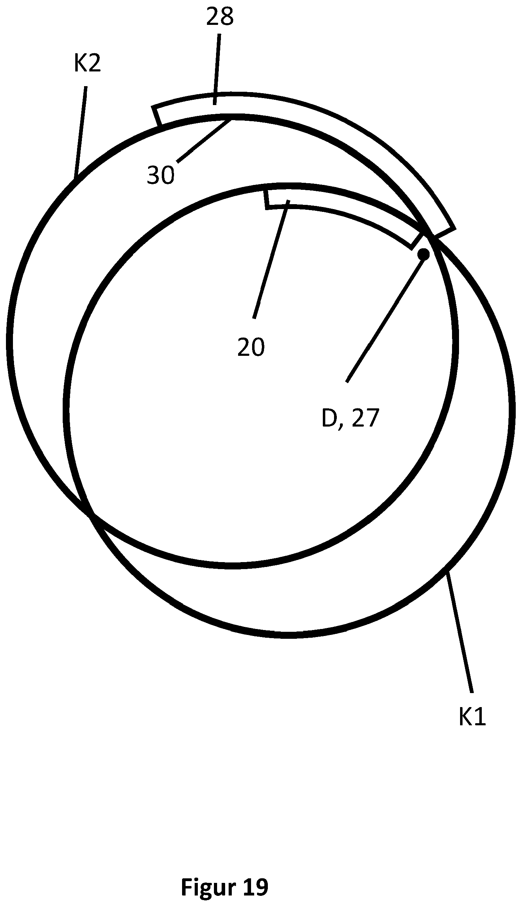

Die Schwenkachse D kann beispielsweise auch in einem Schnittpunkt der beiden Kreise K1 und K2 liegen. In manchen Ausführungsformen liegt die Schwenkachse innerhalb des Kreises K1 und außerhalb des Kreises K2. Eine genauere Darstellung der Kreise in Relation zu Schwenkachse D, Führungsnut 20 und Führungskante 30 ist in Figur 19 dargestellt.

In manchen Ausführungsformen weisen die Führungskanten 30 einen Radius R2 auf, welcher kleiner oder größer ist als der Radius R1 der Führungsnut 20 der Schiene 17. In diesen Ausführungsformen wird insbesondere durch den Radius R2 das Maß der Schwenkbarkeit der Stirnstütze 5 bestimmt. In einigen Ausführungsformen der Stirnstützverstellung ist die Schwenkbarkeit der Stirnstütze durch das Verhältnis des Radius R1 zum Radius R2 bestimmt, wobei der Radius R2 kleiner oder größer sein sollte als der Radius R1. Insbesondere das Verhältnis zwischen R2 und R1 bestimmt dann, in welchem Maße die Stirnstütze schwenkbar ist. Ist R1 größer als R2, liegt das Verhältnis von R2 zu R1 beispielsweise in einem Bereich R2:R1 von 0,5 bis 0,99, bevorzugt in einem Bereich von 0,7 bis 0,99, mehr bevorzugt zwischen 0,75 und 0,985. Je kleiner das Verhältnis von R2 zu R1 ist, desto größer ist der Winkel um welchen die Stirnstütze 5 bei Bewegung des Schiebeelements 10 schwenkt. Eine Bewegung des Schiebeelements 10 führt bei einem kleineren Verhältnis R2:R1 zu einem größeren Schwenkwinkel der Stirnstütze 5 als bei der gleichen Bewegung des Schiebeelements 10 bei einem größeren Verhältnis R2:R1. Ist R2 größer als R1, liegt das Verhältnis von R1 zu R2 beispielsweise in einem Bereich R1:R2 von 0,5 bis 0,99, bevorzugt in einem Bereich von 0,7 bis 0,99, mehr bevorzugt zwischen 0,75 und 0,985. Sind die Radien R1 und R2 unterschiedlich ist für eine Schwenkbarkeit der Stirnstütze ein Schnittpunkt der jeweiligen Kreise K1 und K2 nicht notwendig, soweit die Kreise K1 und K2 keinen gemeinsamen Mittelpunkt aufweisen.

Beispielsweise beträgt der Radius R2 zwischen 7 und 15 cm, bevorzugt zwischen 9 und 11 cm.

In manchen Ausführungsformen weist die Führungskante 30 keine geschwungene Form auf, welcher ein Kreis zugrunde liegt. Beispielsweise kann die Führungskante 30 in einer beliebigen Kurvenform, z.B. parabelförmig, verlaufen oder gerade ausgeführt sein. Die Wahl des Verlaufs der Führungskante 30 ist unabhängig von der Form der Führungsnut 20.

An dem den Pins 27 gegenüberliegendem Ende des Stützarms 6 geht die Verbindung 22 materialmäßig in die Haltermitte 47 des Polsterhalters 9 über. Von der Haltermitte 47 erstreckt sich weiter der Halterrahmen 19 von dem unter anderem die Bänderaufnahmen 41 und die Polsteraufnahmen 38 abgehen. Die Bänderaufnahmen 41 bilden jeweils zusammen mit dem Halterrahmen 19 einen Spalt 36 durch welchen die Kopfbänderung 7 geführt und um die Bänderaufnahme 41 gelegt werden kann.

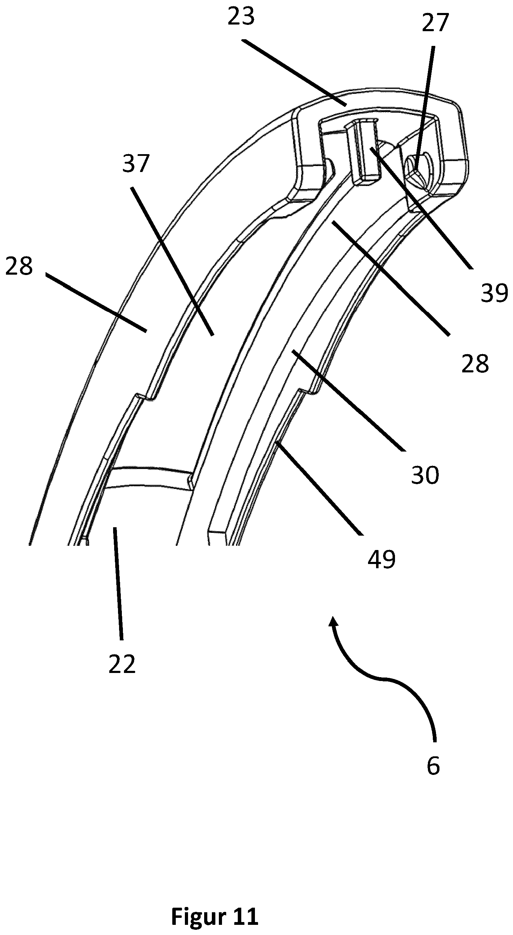

Figur 11 zeigt das Ende des Stützarmes 6 im Detail, an welchem die Pins 27 sowie der Stift 39 angeordnet sind. Die Träger 28 des Stützarms 6 sind über die Verbindungen 22, 23 mit einander verbunden und begrenzen zusammen mit den Trägern 28 den Freiraum 37, welcher das Schiebeelement 10 aufnehmen kann indem die Innenwände 32 des Schiebeelements 10 durch den Freiraum 37 gesteckt werden. An den Kanten 49 der Träger 28, welche den Verbindungen 22, 23 gegenüberliegen, sind beispielhaft geschwungene (bzw. kurvenförmige) Führungskanten 30 ausgebildet. Beispielsweise sind diese Führungskanten 30 innen angeordnet. In manchen Ausführungsformen ist keine extra Führungskante 30 an den Trägern 28 ausgebildet, sondern die Kanten 49 der Träger 28 selbst dienen als Führungskanten 30.

Die Verbindung 23, welche die Träger 28 an dem Ende mit einander verbindet, an welchem die Pins 27 angeordnet sind, weist beispielsweise einen Stift 39 auf. Der Stift 39 erstreckt sich beispielsweise parallel zu den Trägern 28 von der Verbindung 29 in Richtung Maskenkörper 2 und wird vom Zwischenraum 35 des Verbindungselements 16 aufgenommen. Der Stift 39 kann beispielsweise durch das Loch der Verschlusslasche 21 geführt werden um eine unverlierbare Verbindung zwischen Verschluss 15 und Maske 1 herzustellen. In manchen Ausführungsformen weist der Maskenkörper 2 keinen Gasanschluss 18 auf, womit auch der Verschluss 15 nicht notwendig ist. In diesen Ausführungsformen kann beispielsweise auch der Stift 39 am Stützarm 6 nicht ausgebildet sein.

Die Pins 27 sind an einem Ende der Träger 28 bzw. des Stützarms ausgebildet, wobei das Ende, an dem die Pins 27 angeordnet sind, dem Ende gegenüberliegen, an dem Stützarm 6 in den Polsterhalter 9 übergeht. Die Pins 27 stehen beispielsweise senkrecht von den Trägern 28 ab. Beispielsweise sind die Pins 27 nach innen gerichtet, ragen also aufeinander zu, berühren sich aber nicht. In manchen Ausführungsformen sind die Pins 27 außen an den Trägern 28 angeordnet und nach außen gerichtet, weisen also voneinander weg. Auch können statt der Pins 27 Löcher ausgebildet sein. Sind statt Pins 27 Löcher am Ende der Träger 28 ausgebildet, so kann beispielsweise das Verbindungselement 16 über entsprechende Pins verfügen, über welche die Löcher des Stützarms 6 gesteckt werden. Alternativ oder ergänzend kann sowohl das Verbindungselement 16 als auch der Stützarm 6 jeweils über Löcher verfügen. In dem Fall wird zur Verbindung eine Stange/ein Stift durch die Löcher gesteckt. Die Schwenkachse D der Stirnstütze 5 wird über die Pins 27 des Stützarms 6 definiert

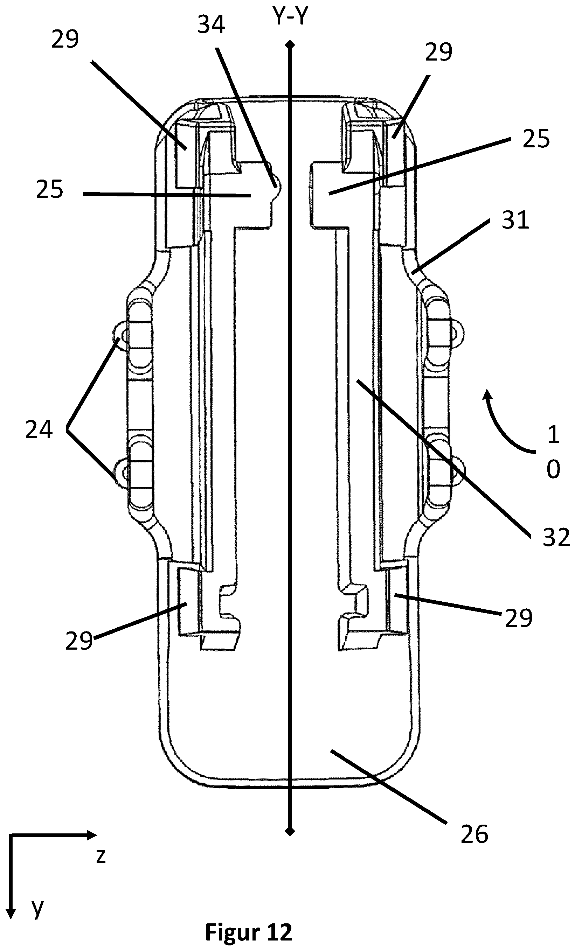

Eine beispielhafte Ausführungsform des Schiebeelemente 10 ist schematisch in den Figuren 12 bis 15 dargestellt. Die in den Figuren 12 bis 15 eingezeichneten Koordinatensysteme beziehen sich ausschließlich auf die Figuren 12 bis 15 und stimmen nicht genau mit den Koordinatensystemen der anderen Figuren überein.

Figur 12 zeigt eine Unteransicht des Schiebeelements 10, also auf die Unterseite der Schieberplatte 26. Auf der Unterseite der Schieberplatte 26 sind zwei Innenwände 32 ausgebildet, welche im Wesentlichen senkrecht zu der Schieberplatte 26 stehen und parallel zueinanderstehen. An den Innenwänden 32 sind die Führungselemente 29 sowie die Zapfen 25 angeordnet, vorzugsweise an der Kante der Innenwände 32, welche der Schieberplatte 26 gegenüberliegen. Die Zapfen 25 sind beispielsweise an einer der Ecken der Innenwände 32 angeordnet, beispielsweise nach innen gerichtet, also aufeinander zuweisend. An zumindest einem der Zapfen 25 ist zusätzlich eine Rastnase 34 ausgebildet, welche in den Einkerbungen 33 der Schiene 17 einrasten kann um die Schiebebewegung des Schiebeelements 10 einzuschränken beziehungsweise zu fixieren, sodass wahlweise diskrete Einstellstufen der Stirnstütze 5 gewählt werden können.

In manchen Ausführungsformen weist das Schiebeelement 10 lediglich eine Innenwand auf, an welcher zum Beispiel beidseitig jeweils ein Zapfen 25 aufweist und auf zumindest einer Seite Führungselemente 29. Auch ist es für manche Ausführungsformen möglich, dass eine dritte Innenwand 32 parallel zwischen den beiden Innenwänden 32 angeordnet ist. Beispielsweise kann diese Innenwand zusätzliche Führungselemente 29 und/oder Zapfen 25 aufweisen. Entsprechend dazu müssten beispielsweise weitere Schienen 17 bzw. Führungsnuten 20 und/oder auch Führungskanten 30 angeordnet werden.

An der gleichen Kante der Innenwände 32, an der die Zapfen 25 angeordnet sind, sind die Führungselemente 29 ausgebildet. Beispielsweise ist an jeder Ecke jeweils ein Führungselement 29 angeordnet. Die Führungselemente 29 sind beispielsweise so angeordnet, dass sie von der Innenwand 32 ausgehend nach außen zeigen, also voneinander ausgerichtet sind. Zumindest auf eine Seite, bevorzugt die Seite, welche zur Schieberplatte 26 zeigt, ist weitestgehend plan ausgeführt, wobei die so dargestellte Fläche in manchen Ausführungsformen mit einem Radius R3 gekrümmt ist. Der Radius R3 entsprich in manchen Ausführungsformen beispielsweise dem Radius R2 der Führungskante 30. In manchen Ausführungsformen sind die Führungselemente 29 so eingerichtet, dass diese auf den Verlauf der Führungskante 30 angepasst sind. In manchen Ausführungsformen sind die Führungselemente 29 rund ausgeführt, sodass eine minimale Kontaktfläche zwischen Führungselement 29 und Führungskante 30 entsteht.

In manchen Ausführungsformen sind die Führungselemente 29 nach innen ausgerichtet und an der Innenseite der Innenwände 32 ausgebildet. Dementsprechend sind in manchen Ausführungsformen die Zapfen 25 an der Außenseite der Innenwände 32 und nach außen gerichtet angeordnet. In manchen Ausführungsformen sind Zapfen 25 und Führungselemente 29 auf der gleichen Seite der jeweiligen Innenwand 32 angeordnet. In dem Falls sollten die Führungselemente 29 oberhalb, in Richtung Schieberplatte 26 versetzt, der Zapfen 25 angeordnet sein, sodass der Stützarm 6 oberhalb der Schiene 17 angeordnet und geschwenkt werden kann.

Statt zwei einzelner Führungselemente 29 je Innenwand 32 kann in manchen Ausführungsformen auch ein zusammenhängendes Führungselement je Innenwand 32 ausgebildet sein, welches sich über die gesamte oder zumindest einen Großteil (>50%) der Länge (y-Richtung in Figur 12) der Innenwand 32 erstreckt.

An den äußeren (z-Richtung) Kanten der Schieberplatte 26 geht die Schieberplatte in die Flügelelemente 31 über. An den Flügelelementen 31, welche in manchen Ausführungsformen in zumindest zwei Unterflügel 44 übergehen, sind Griffstrukturen 24 angeordnet, welche eine höhere Griffigkeit des Schiebeelements 10 bewirken. Die Flügelelemente 31 stehen dabei im Wesentlichen senkrecht zu der Schieberplatte 26 und verlaufen parallel zu den Innenwänden 32.

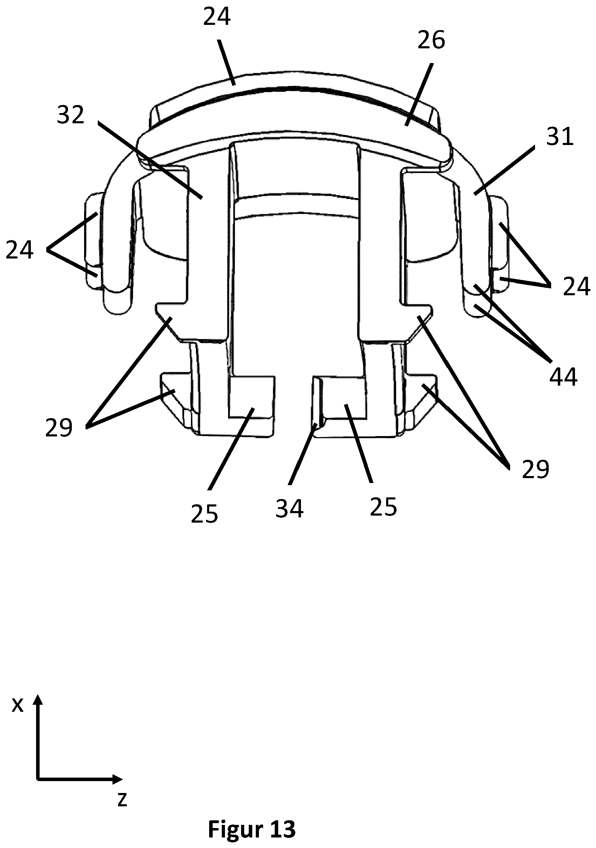

Eine Frontansicht (entlang der y-Achse in Figur 12) einer beispielhaften Ausführungsform des Schiebeelements 10 ist in Figur 13 dargestellt. Auf der Schieberplatte 26 sowie an den Seiten der Flügelelemente 31 sind beispielsweise Griffstrukturen 24 angeordnet, welche eine erhöhte Griffigkeit des Schiebeelements 10 bewirken. Von der Schieberplatte 26 gehen zwei zu einander parallel verlaufende Innenwände 32 ab. Die Innenwände 32 stehen im Wesentlichen senkrecht zu der Schieberplatte 26, wobei die Schieberplatte 26 in manchen Ausführungsformen eine Krümmung aufweist. Parallel zu den Innenwänden 32 verlaufen die Flügelelemente 31, in welche die Schieberplatte 26 zu den Seiten übergeht.

Sowohl im vorderen Bereich, als auch im hinteren Bereich der Innenwände 32 sind nach außen weisend die Führungselemente 29 angeordnet. Die Führungselemente 29 stehen dabei senkrecht zu den Innenwänden 32 ab. Beispielsweise sind die Führungselemente 29 auf der Seite, welche zu der Schieberplatte 26 weist, flach ausgebildet, sodass die Führungskante 30 aufliegen kann. Die Führungselemente 29 sind beispielsweise nach unten hin (x-Richtung) abgewinkelt ausgebildet, sodass ein vereinfachtes Stecken in bzw. durch den Freiraum 37 der Stirnstütze 5 ermöglicht wird. Die Träger 28 des Stützarms 6 werden beispielsweise zwischen Schieberplatte 26 und Führungselementen 29 beweglich eingeklemmt. Ein Verschieben der Schiebelements 10 entlang des Stützarms 6 bleibt so möglich, während ein versehentliches Lösen von Stützarm 6 und Schiebeelement 10 verhindert wird. Auch ist ein Schwenken der Stirnstütze 5 ohne gleichzeitige Drehbewegung des Schiebeelements 10 nicht möglich, die Orientierung des Schiebeelements 10 zum Stützarm 6 bleibt dabei immer gleich.

Im hinteren Bereich der Innenwände 32 ist nach innen gerichtet jeweils ein Zapfen 25 angeordnet, welche in die Führungsnut 20 der Schiene 17 aufgenommen werden können. Die Zapfen 25 sind so ausgebildet, dass diese drehbar und schiebbar in der Führungsnut 20 gelagert sind. Zumindest einer der Zapfen 25 weist eine Rastnase 34 auf, welche in den Einkerbungen 33 der Schiene 17 einrasten kann um die Einstellung der Stirnstütze gegenüber einem versehentlichen Verstellen zu fixieren.

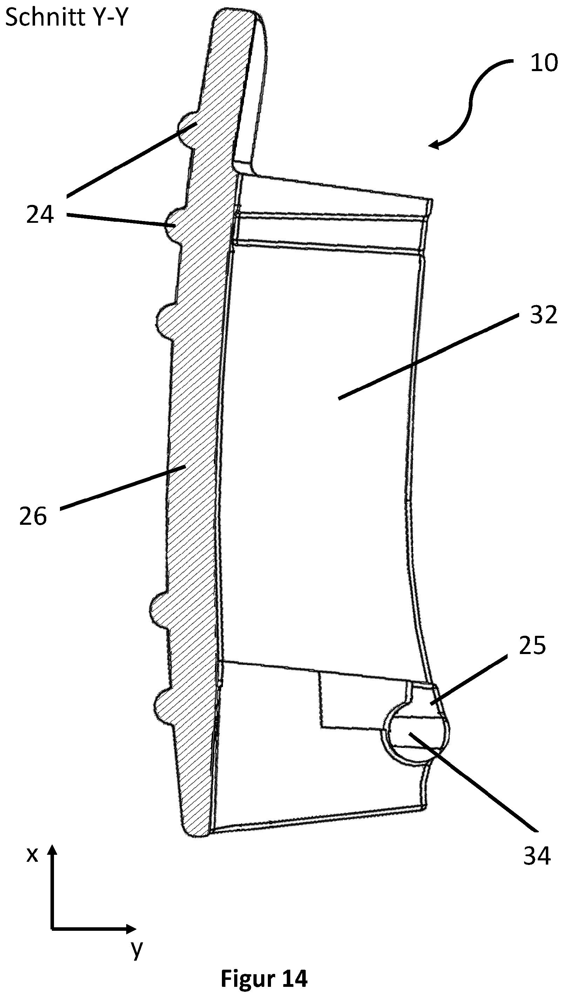

Die Figur 14 zeigt einen Schnitt durch eine beispielhafte Ausführungsform des Schiebeelements 10 entlang der Schnittkante Y-Y (Figur 12). Die Schiebeplatte 26 ist beispielhaft leicht geschwungen/bogenförmig ausgebildet, wobei beispielsweise ein Bogen beschrieben wird, welcher in etwa dem Radius R2 der Führungskante 30 entspricht. Dabei kann der Radius der Schieberplatte 26 geringfügig (+/- 10%) von dem Radius R2 abweichen. In manchen Ausführungsformen ist die Schieberplatte 26 auch eben/plan ausgeführt. Auf der Schieberplatte 26 sind beispielsweise Griffstrukturen 24 angeordnet.

In manchen Ausführungsformen ist die Schieberplatte 26 beispielsweise entsprechend der Träger 28 geformt. Verlaufen die Träger 28 beispielsweise gerade oder parabelförmig, so weist die Schieberplatte 26 ebenfalls eine gerade oder parabelförmige Form auf, zumindest aber die Unterseite der Schieberplatte 26 folgt in den Bereichen, in denen sie auf dem Träger 28 aufliegt, im Wesentlichen der Form des Trägers 28.

Im Wesentlichen senkrecht zu der Schieberplatte 26 erstreckt sich auf der den Griffstrukturen 24 gegenüberliegenden Seite der Schieberplatte 26 die Innenwand 32. In der in Figur 14 dargestellten Ansicht wird auf die Innenseite einer der Innenwände 32 geblickt. In einer der Ecken der Innenwand 32, an der Kante, welcher der Schieberplatte 26 gegenüberliegt, ist ein Zapfen 25 ausgebildet, welcher in die Führungsnut 20 der Schiene 17 aufgenommen wird um eine dreh- und schiebbare Lagerung des Schiebeelements 10 in der Schiene 17 zu erreichen. Beispielsweise ist an dem Zapfen 25 eine Rastnase 25 ausgebildet, welche in den Einkerbungen 33 der Führungsnut 20 beziehungsweise der Schiene 17 einrasten kann.

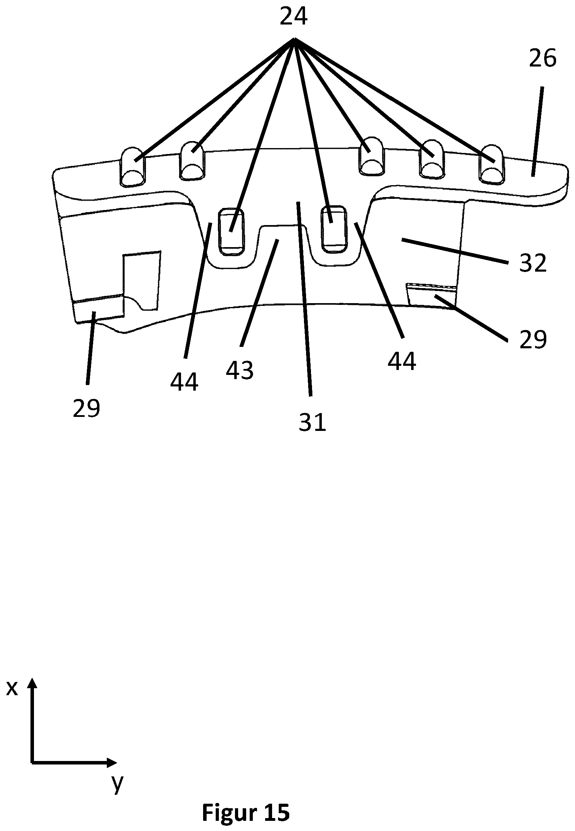

Eine Seitansicht einer beispielhaften Ausführungsform des Schiebeelements 10 ist in Figur 15 a) dargestellt. Die Schieberplatte 26 beispielsweise in den Flügel 31 über, welcher parallel zu der Innenwand 32 verläuft. Der Flügel 31 weist beispielhaft zwei Unterflügel 44 auf, auf denen jeweils eine Griffstruktur 24 angeordnet ist. Zwischen den Unterflügeln 44 befindet sich ein Zwischenraum 43. In manchen Ausführungsformen sind auf der Außenseite des Stützarms 6 diskrete Bezeichnungen (beispielsweise eine Nummerierung und/oder Wertangaben wie z.B. Schwenkwinkel) für die Schwenkstellungen und/oder zur Markierung der Schwenkstellung angeordnet, zum Beispiel durch Aufdrucken. Eine Schwenkstellung kann beispielsweise dadurch gekennzeichnet sein, dass das Schiebelement 10 in der entsprechenden Stellung mit der Rastnase 34 in einer Einkerbung 33 einrasten kann. Diese Bezeichnungen sind beispielsweise so angeordnet, dass die der Schwenkstellung entsprechenden Bezeichnung jeweils zwischen den Unterflügeln 44 in dem Zwischenraum 43 zu sehen sind.

Die zwei in Figur 15 dargestellten Führungselemente 29 sind beispielhaft leicht gewinkelt zu einander angeordnet. Durch die zueinander gewinkelten Führungselemente 29 wird im Wesentlichen dem bogenförmigen/geschwungenen Verlauf der Führungskante 30 entsprochen. In manchen Ausführungsformen sind die Führungselemente 29 selbst geschwungen ausgeführt, sodass der beschriebene Bogen einen Radius R3 aufweist, welcher dem Radius R2 der Führungskante 30 im Wesentlichen entspricht.

Die Führungselemente 29 sind beispielhaft mit einer länglichen (in y-Richtung der Figur 15) Ausdehnung ausgebildet. In manchen Ausführungsformen können die Führungselemente 29 auch eine in Aufsicht (entlang der Sichtachse in Figur 15) im Wesentlichen runde Form aufweisen.

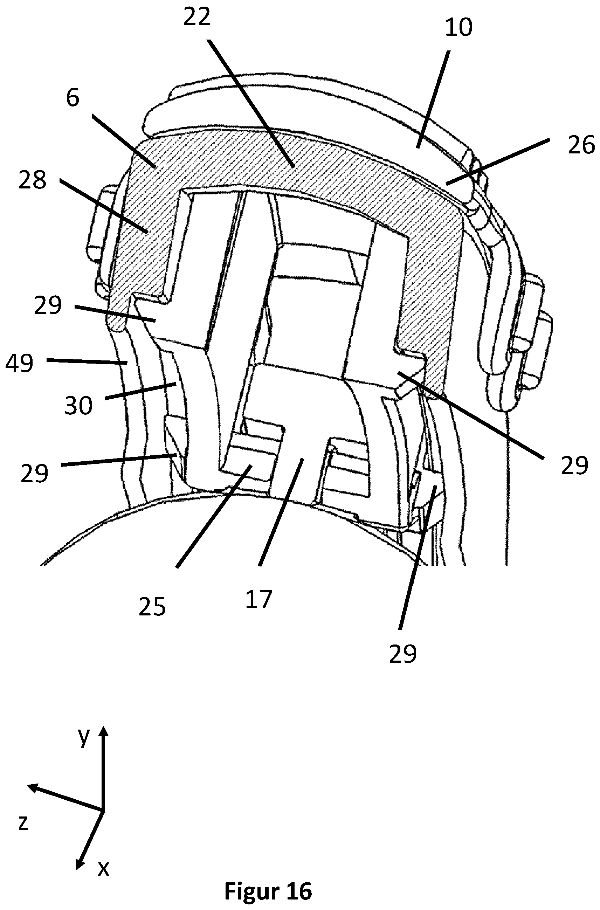

Ein Schnitt durch eine beispielhafte Ausführungsform des Stützarms 6 in einer beispielhaften Anordnung mit Schiene 17 auf Maskenkörper 2 und eingesetztem Schiebeelement 10 ist in Figur 16 zu sehen. Die Schnittebene durch den Stützarm 6 ist unmittelbar nach Beginn der Verbindung 22 im Anschluss zum Freiraum 37 gesetzt. In der dargestellten Ansicht befindet sich das Schiebelement 10 beispielhaft mit der Rastnase 34 an einem der Zapfen 25 in der letzten Einkerbung der Schiene 17, das heißt in der von dem Verbindungselement 16 entferntesten Position. Ein weiteres Verschieben des Schiebeelements 10 von dem Verbindungselement 16 weg wird durch die Verbindung 22 verhindert, an welche das Schiebelement 10 mit den Innenwänden 32 anstößt. In dieser Einstellung liegt die Stirnstütze 5 am engsten an dem Maskenkörper 2 an. Wird das Schiebeelement 10 in Richtung Verbindungselement 16 geschoben, so wird der Stützarm 6 um die Schwenkachse D, welche beispielsweise durch die Pins 27 im Verbindungselement 16 definiert wird, geschwenkt.

Die Zapfen 25 des Schiebeelements 10 sind in der Führungsnut 20 der Schiene 17 dreh- und schiebbar gelagert, können also entlang der Schiene 17 verschoben und dabei gedreht werden. Die Träger 28 des Stützarms 6 liegen beispielhaft mit der Führungskante 30 auf den Führungselementen 29 des Schiebeelements 10 auf beziehungsweise sind zwischen der Schieberplatte 26 und den Führungselementen 29 geklemmt. Je nach Bewegungsrichtung des Schiebeelements 10 drücken entweder die Schieberplatte 26 von oben auf die Träger 28 oder die Führungselemente 29 von unten auf die Führungskante 30 um eine Schwenkbewegung der Stirnstütze 5 um die Schwenkachse D zu bewirken. Die Flügelelemente 31 des Schiebeelements 10 sind so am Schiebeelement 10 angeordnet, dass diese außen (in z-Richtung) an den Seiten der Träger 28 angeordnet sind. Die Innenwände 32 des Schiebeelements 10 befinden sich beispielsweise auf der den Flügelelementen 31 gegenüberliegenden Seite der Träger 28.

Wird das Schiebelement 10 in Richtung Verbindungselement 16 geschoben, so wird das Schiebelement 10 entlang der Führungsnut 20 der Schiene 17 geführt. Da die Kreise der Führungskante 30 des Stützarms 6 und der Führungsnut 20 keinen gemeinsamen Mittelpunkt aufweisen beziehungsweise die Konturen der Führungsnut 20 und der Führungskante 30 nicht parallel verlaufen, treffen bei der Bewegung die Führungselemente 29 auf die Träger 28 bzw. die Führungskante 30. Da der Stützarm 6 schwenkbar im Verbindungselement 16 gelagert ist, kann ein Schwenken des Stützarms 6 erfolgen, wodurch dem Druck der Führungselemente 29 des Schiebeelements 10 nachgegeben wird. Die Träger 28 wiederum üben durch die Schwenkbewegung der Stirnstütze 5 einen Druck auf die Schieberplatte 26 aus. Um diesem Druck nachzugeben dreht sich das gesamte Schiebeelement 10 in der Führungsnut 20. Bei der Schiebebewegung des Schiebeelements 10 in Richtung des Verbindungselements 16 schwenken bzw. drehen also gleichzeitig das Schiebeelement 10 sowie die Stirnstütze 5. Die Schwenk-/Drehachse des Schiebeelements 10 verläuft durch die beiden Zapfen 25.

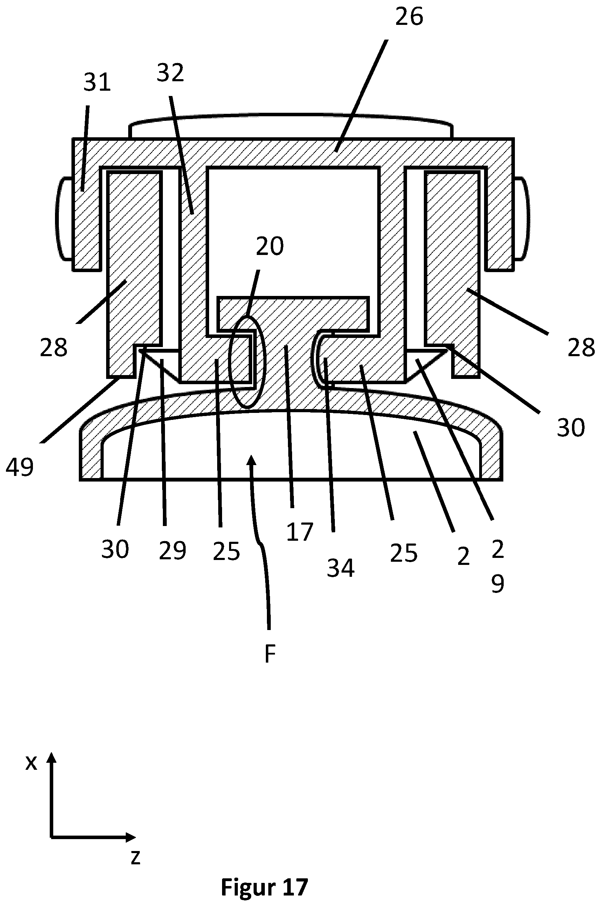

Figur 17 zeigt einen Schnitt durch eine beispielhafte Ausführungsform der Stirnstützverstellung bestehend aus Schiebeelement 10, Stirnstütze 5 und Schiene 17. Die Träger 28 sind zwischen Schieberplatte 26 und Führungselementen 29 eingeklemmt, wobei die Führungskante 30 auf den Führungselementen 29 aufliegt. In manchen Ausführungsformen ist an den Trägern 28 keine spezielle Führungskante 30 ausgebildet, sodass die untere Kante 49 des Trägers 28 selbst die Führungskante 30 darstellt.

Die Zapfen 25 des Schiebeelements 10 verlaufen in der Führungsnut 20 der Schiene 17. An einer der Zapfen 25 ist beispielhaft eine Rastnase 24 angeordnet, welche in den Einkerbungen 33 der Schiene 17 einrasten kann und damit eine unbeabsichtigte Schiebebewegung des Schiebeelements 10 verhindert und die Stirnstütze 5 in einer Schwenkstellung fixiert. An den Seiten der Träger 28 erstrecken sich senkrecht von der Schieberplatte 26 ausgehend die Flügelelemente 31 sowie die Innenwände 32.

Beispielhaft sind die Zapfen 25 und die Führungselemente 29 auf gleicher Höhe (x-Richtung in Figur 17) an den Innenwänden 32 des Schiebeelements 10 angeordnet. In manchen Ausführungsformen können die Zapfen 25 und die Führungselemente 29 auch auf unterschiedlichen Höhen angeordnet sein. Auch die Anordnung an der Außenseite (Führungselemente 29) und Innenseite (Zapfen 25) der Innenwände 32 kann in manchen Ausführungsformen verschieden sein. In manchen Ausführungsformen ist die Stirnstützverstellung beispielsweise so aufgebaut, dass die Träger 28 mit den Führungskanten 30 mittig, oberhalb der Schiene 17 verlaufen. In dem Fall wären sowohl Führungselemente 29 als auch Zapfen 25 an den Innenseiten der Innenwände 32 anzuordnen. Die Zapfen 25 sind jeweils auf der Seite der Innenwände 32 anzuordnen an welcher die Schiene 17 mit der Führungsnut 20 verläuft. Die Führungselemente 29 sind jeweils auf der Seite anzuordnen, auf denen die Führungskante 30 verläuft.

Die Schiene 17 ist beispielhaft materialmäßig mit dem Maskenkörper 2 verbunden. Beispielsweise werden Maskenkörper 2 und Schiene 17 einteilig gefertigt. In manchen Ausführungsformen kann die Schiene 17 aber auch extra gefertigt werden und auf den Maskenkörper 2 aufgesetzt und durch Stecken, Klemmen, Kleben und/oder Schrauben oder ähnliche Verbindungsmethoden mit dem Maskenkörper 2 verbunden werden.

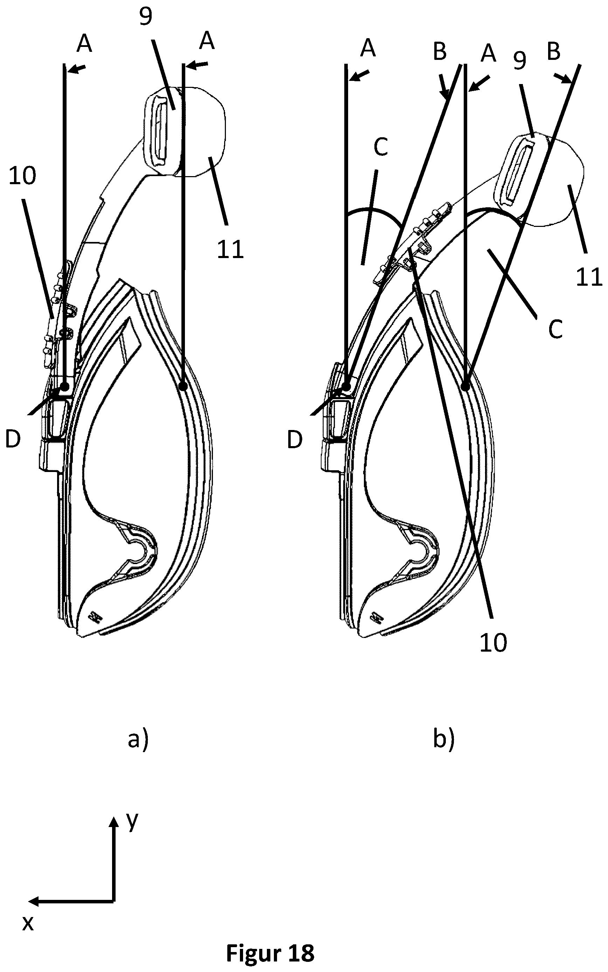

Der beispielhafte Schwenkbereich der Stirnstütze 5 ist in Figuren 18 a) und b) dargestellt. In Figur 18 a) befindet sich das Schiebeelement 10 in der Rastposition, welche dem Verbindungselement 16 und damit der Schwenkachse D am nächsten liegt. Die Rastnase 34 ist also in der Einkerbung 33, welcher dem Verbindungselement am nächsten liegt, eingerastet. In dieser Stellung lässt sich beispielsweise zwischen Polsterhalter 9 und Stirnpolster 11 eine Ebene A definieren, welche im Wesentlichen durch die Fläche des Polsterhalters 9 definiert wird. Diese Ebene A lässt sich parallel so verschieben, sodass die Schwenkachse D auf der Ebene A liegt.

In Figur 18 b) befindet sich das Schiebeelement 10 in der Rastposition welche dem Verbindungselement 16 und damit der Schwenkachse D am weitesten entfernt liegt. Betrachtet man hier die Ebene B, welche beispielsweise anhand der Fläche des Polsterhalters 9 definiert werden kann, so ist diese gegenüber der Ebene A um den Winkel C gedreht. Der Winkel C stellt beispielsweise den maximalen Schwenkwinkel der Stirnstütze 5 um die Schwenkachse D dar. Beispielsweise liegt der maximale Schwenkwinkel C der Stirnstütze 5 in einem Bereich von 10° bis 40°, vorzugsweise zwischen 15° und 25°.

Der maximale Schwenkwinkel C gibt dabei die Veränderung des Winkels an, welcher zwischen den jeweiligen ersten und letzten Schwenkstellungen ergibt. Beispielsweise kann der Schwenkwinkel, bei dem das Schiebeelement 10 in der dem Verbindungselement 16 am nächsten liegenden Rastposition sitzt als 0° definiert werden. In der vom Verbindungselement 16 am weitesten entfernten Rastposition nimmt der Schwenkwinkel dann den Wert des maximalen Schwenkwinkels C an.

Der maximale Schwenkwinkel C hängt dabei von mehreren Faktoren ab. Insbesondere beeinflusst die Strecke, über welche das Schiebeelement 10 bewegt werden kann, den maximalen Schwenkwinkel. Je länger diese Strecke gewählt wird, desto größer wird der Schwenkwinkel C. Die Strecke in der Führungsnut 20, um welche das Schiebeelement 10 verschoben werden kann, beträgt zum Beispiel zwischen 2 cm und 10 cm, bevorzugt zwischen 2,5 cm und 5 cm, mehr bevorzugt zwischen 2,5 cm und 4 cm.

Zudem wird der maximale Schwenkwinkel durch das Verhältnis des Radius R2 der Führungskante 30 zum Radius R1 der Führungsnut 20 bestimmt. Beispielsweise nimmt der maximale Schwenkwinkel C bei gleichbleibender Strecke für das Schiebeelement 10 zu, wenn das Verhältnis R2:R1 abnimmt. Wird die Strecke, um welche das Schiebeelement 10 verschoben werden kann, verlängert, nimmt bei gleichbleibendem Verhältnis R2:R1 der maximale Schwenkwinkel C zu.

Beim Schwenken der Stirnstütze 5 um die Schwenkachse D ändert sich die Positionierung des Polsterhalters 9 und damit auch des Stirnpolsters 11 relativ zum Maskenkörper 2. Auch ändert sich dabei die Orientierung des Stirnpolsters 11 um den Winkel C. Anhand Figur 18 a) und b) beschrieben, bewegt sich beim Schwenken der Stirnstütze 5 das Stirnpolster entgegen der x-Richtung und entgegen der y-Richtung. Im Wesentlichen wird die Stirnstütze 5 von Figur 18 a) zu Figur 18 b) näher an den Maskenkörper 2 geführt.

Wird das Schiebelement 10 entlang der Schiene 17 in Richtung Polsterhalter 9 bewegt, wie beispielsweise von der Position in Figur 18 a) zur Position in Figur 18 b), so nähert sich der Stützarm 6 dem Maskenkörper 2 an, bis die maximale Schwenkposition erreicht ist.

Geht man davon aus, dass die Maske an einem Gesicht anliegt, wobei die Stirnstütze 5 mit einer Kopfbänderung an der Stirn des Anwenders fixiert ist, so würde ein Verstellen der Stirnstütze 5 eine Veränderung der Positionierung des Maskenkörpers 2 bewirken. Beispielsweise wird der obere Bereich des Maskenkörpers 2 durch ein Verschieben des Schiebeelements 10 in die Rastposition, welche am entferntesten von dem Verbindungselement 16 ist, von dem Gesicht des Anwenders weggedrückt.

In Figur 19 ist eine beispielhafte Lage der Kreise K1 und K2 mit der auf dem jeweiligen Umfang verlaufenden Führungsnut 20 und Führungskante 30 zueinander beispielhaft schematisch dargestellt. Die dargestellte Lage ist beispielsweise nur für eine Schwenkposition gültig. Die Kreise K1 und K2 weisen Radien R1 und R2 auf, welche beispielhaft gleich groß sind. Die Mittelpunkte der Kreise K1 und K2 sind versetzt zueinander, sodass sich die Kreise schneiden. Die Führungsnut 20 verläuft entlang eines Teilstückes des Umfangs des Kreises K1. Die Führungskante 30 verläuft entlang eines Teilstückes des Umfangs des Kreises K2. Beispielsweise weisen sowohl Führungskante 30 als auch Führungsnut 20 ein Ende im Bereich des Schnittpunktes der Kreise K1 und K2 auf. Die Schwenkachse D, welche beispielsweise durch die Pins 27 definiert wird, befindet sich in der gezeigten beispielhaften Ausführungsform innerhalb der Kreise K1 und K2. Beim Verschieben des Schiebeelements 10 dreht der Kreis K2 um die Schwenkachse D während der Kreis K1 fest bleibt. Dadurch schwenkt die Führungskante 30 relativ zu der Führungsnut 20 um die Schwenkachse D. In manchen Ausführungsformen kann die Schwenkachse D frei angeordnet werden. So ist eine Anordnung außerhalb K1 aber innerhalb K2, außerhalb sowohl K1 als auch K2 und auch eine Anordnung auf dem Umfang von K1 und/oder K2 möglich. Beim Schwenken des Trägers 28 um die Schwenkachse D verändert sich gleichzeitig auch die Lage der Kreise K1 und K2 zueinander. Dass die Kreise K1 und K2 in eine deckungsgleiche Lage kommen oder die Kreise parallel zu einander verlaufen wird beispielsweise dadurch verhindert, dass der Abstand J zwischen den Führungselementen 29 und den Zapfen 25 nicht dem Abstand K zwischen der Oberkante 51 Führungsnut 20 und der Führungskante 30 entspricht, wenn die Führungsnut 20 und die Führungskante 30 durch ein Schwenken um die Schwenkachse D parallel zu einander gestellt sind.

Ob die Schwenkachse D innerhalb oder außerhalb des Kreises K2, welcher um die Schwenkachse D drehbar ist, hängt primär von der aktuellen Schwenkposition ab. Im Zusammenhang mit dem maximalen Schwenkwinkel C kann aber erreicht werden, dass die Schwenkachse D immer außerhalb oder innerhalb des Kreises K2 liegt. Auch kann so beispielsweise erreicht werden, dass bei einem Schwenken die Lage der Schwenkachse D relativ zum Kreis K2 von innerhalb zu außerhalb bzw. umgekehrt wechselt.

Auch bei einem geraden oder parabelförmigen Verlauf der Führungsnut 20 und der Führungskante 30 sollte der Abstand J zwischen Führungselementen 29 und den Zapfen 25 nicht dem Abstand K zwischen Oberkante 51 der Führungsnut 20 und der Führungskante 30 in paralleler Stellung entsprechen, da ansonsten keine Schwenkbewegung durch das Verschieben des Schiebelements 10 erreicht werden kann. Bevorzugt ist der Abstand J zwischen Führungselementen 29 und Zapfen 25 größer als der Abstand K zwischen Oberkante 51 der Führungsnut 20 und der Führungskante 30, wenn diese durch eine Schwenkstellung parallel zueinander verlaufen.

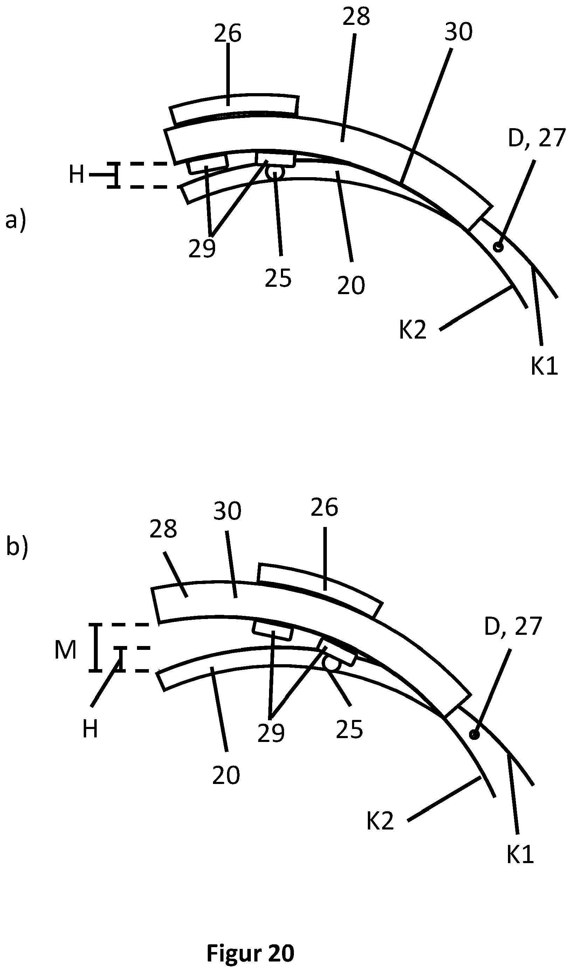

In Figur 20 ist die Funktionsweise der einer beispielhaften Ausführungsform der Stirnstützverstellung schematisch dargestellt, wobei von dem Schiebeelement 10 nur die Zapfen 25, die Schieberplatte 26 sowie die Führungselemente 29 dargestellt sind. Beispielhaft ist eine Ausführungsform gezeigt, bei der sowohl Führungsnut 20 als auch Führungskante 20 und Träger 28 geschwungen und einem Kreisumfang folgend ausgebildet sind. Der Radius R2 ist beispielhaft gleich dem Radius R1, die Kreise K1 und K2 schneiden sich beispielhaft und die Schwenkachse D liegt beispielsweise außerhalb von Kreis K2 und innerhalb von Kreis K1.

Der Verlauf der Kreise K1 und K2 ist beispielhaft durch die Verlängerungen, welche mit K1 und K2 bezeichnet sind, angedeutet. Das Schiebeelement 10 umfasst unter anderem die Schieberplatte 26, die Zapfen 25 sowie die Führungselemente 29, welche in einem festen Abstand zu einander angeordnet sind. Dies wird beispielsweise dadurch erreicht, wenn das Schiebeelement 10 einteilig hergestellt wird.

Wird das Schiebeelement 10 in die Richtung der Schwenkachse D, welche beispielhaft durch die Lage der Pins 27 definiert wird, verschoben, werden die Zapfen 25 entlang der Führungsnut 20 geführt. Die Führungselemente 29 drücken dabei auf die Führungskanten 30 der Träger 28 (Teil des Stützarms 6). Dadurch wird der Stützarm 6, um die Schwenkachse D geschwenkt. Der Träger 28 ist zwischen der Schieberplatte 26 und den Führungselementen 29 geklemmt, wobei die Anordnung der Schieberplatte 26 und der Führungselemente 29 auf die Kontur der Träger 28 angepasst ist. Um beim Schwenken der Träger 28 dieser Kontur weiter zu folgen bewegen sich die Führungselemente 29 und die Schieberplatte 26 entlang des Trägers. Dadurch dreht sich das Schiebeelement 10 gleichzeitig um die Zapfen 25.

Wird das Schiebeelement 10 von der Schwenkachse D weggeschoben, so drückt beispielsweise die Schieberplatte 26 auf die Träger 28. Daraus resultierend findet eine Drehbewegung des Schiebeelements 10 um die Zapfen 25 statt.

Das Schiebeelement 10 ist dazu ausgebildet, sowohl dem Verlauf des Kreises K1, also der Führungsnut 20, als auch dem Verlauf des Kreises K2, also der Führungskante 30 und/oder dem Träger 28, zu folgen. Beide Kreise verlaufen dabei nicht deckungsgleich, sodass beispielsweise bei einer Bewegung des Schiebeelements 10 entlang der Führungsnut 20 gleichzeitig eine Veränderung der der Orientierung des Schiebeelements 10 durch eine Drehung um die Zapfen 25 notwendig ist, damit das Schiebeelement 10 beiden, von K1 und K2, Verläufen folgen kann.

In manchen Ausführungsformen kann beispielsweise auch eine Bewegung des Schiebeelements 10, also eine Verschiebung mit gleichzeitiger Drehung um die Zapfen 25, dadurch erreicht werden, wenn die Stirnstütze 5 um die Schwenkachse D geschwenkt wird. Dabei wird, je nach Schwenkrichtung, durch den Träger 28 Druck auf die Schieberplatte 26 oder die Führungselemente 29 ausgeübt, welche dann bestrebt sind der Kontur der Träger 28 beziehungsweise der Führungskante 30 zu folgen.

In der Position a) der Figur 20 hat die Führungskante 30 an einem Ende einen Abstand H zu der Führungsnut 20. Wird das Schiebeelement 10 in Richtung der Schwenkachse D geschoben (Position b) der Figur 20), so schwenkt der Stützarm 6, wodurch sich der Abstand der Führungskante 30 zu der Führungsnut 20 auf den Abstand M erhöht. Durch das Verschieben des Schiebeelements 10 in Richtung Schwenkachse D erhöht sich also der Abstand zwischen Träger 28 und Führungsnut 20, also auch von dem Maskenkörper 2.

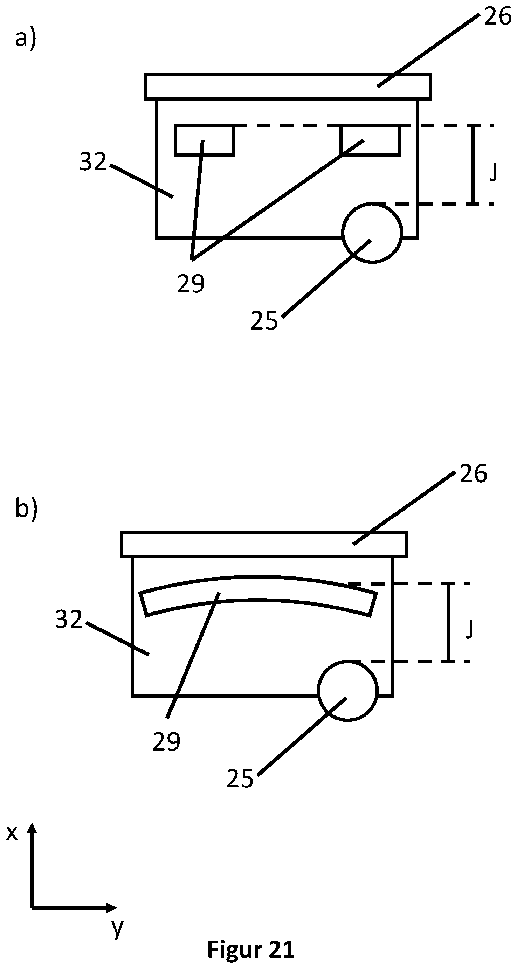

In Figur 21 a) ist eine stark vereinfachte, schemenhafte Darstellung einer beispielhaften Ausführungsform des Schiebeelements 10 gezeigt. Hier soll insbesondere auf den Abstand J zwischen Oberkante (in x-Richtung) der Führungselemente 29, welche in gleichem Abstand J zum Zapfen 25 angeordnet und gerade ausgeführt sind, sowie dem obersten Punkt des Zapfens 25 hingewiesen werden. Der Abstand J ist bevorzugt nicht gleich dem Abstand K zwischen Oberkante 51 der Führungsnut 20 und der Führungskante 30, wenn durch Schwenken in eine Position gebracht werden in welcher Führungsnut 20 und Führungskante 30 parallel zueinander verlaufen oder, im Falle von geschwungenen Formen mit gleichen Radius, deckungsgleich sind.

In Figur 21 b) ist ein einzelnes Führungselement 29 ausgebildet, welches beispielhaft geschwungen und mit dem Radius R3 ausgeführt ist, wobei der Radius R3 beispielhaft dem Radius R2 der Führungskante 30 entspricht. Bei einer solchen Ausführung ist der Abstand J zwischen dem höchsten Punkt in x-Richtung des Zapfens 25 und dem in x-Richtung genau darüber liegenden Punkt auf dem Führungselement 29.

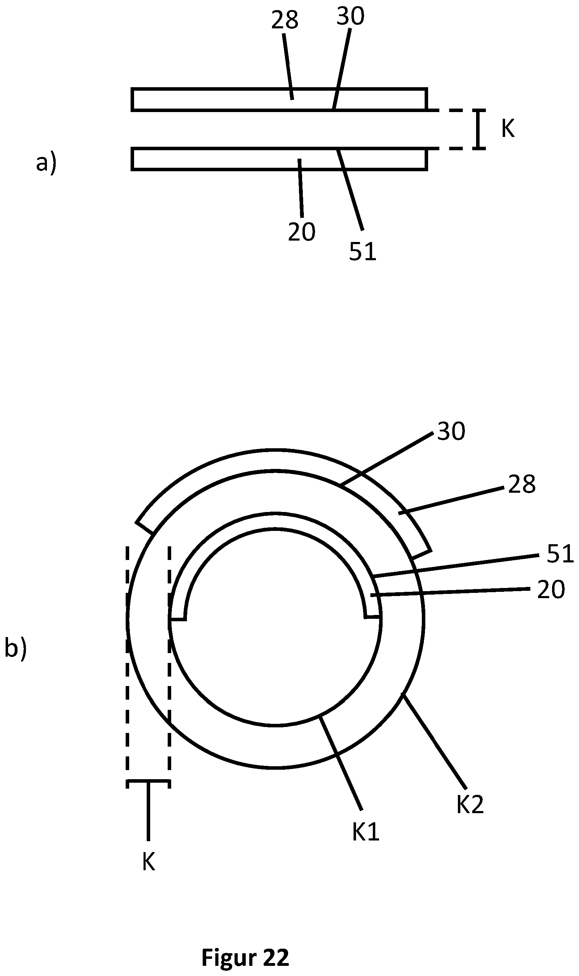

Figur 22 a) und b) stellt beispielhaft zwei Fälle dar, in denen die Führungsnut 20 und die Führungskante 30 durch Schwenken um die Schwenkachse (nicht dargestellt, die genaue Lage der Schwenkachse ist hier unerheblich) in einen parallelen Verlauf gebracht wurden. In Figur 21 a) sind Führungsnut 20 und Führungskante 30 gerade ausgeführt und verlaufen in einem Abstand K parallel zueinander. Der Abstand J (vgl. Figur 21 a)) zwischen Führungselementen 29 und Zapfen 25 sollte bevorzugt größer sein als der Abstand K.

Die Führungsnut 20 und die Führungskante 30 folgen in Figur 22 b) einem geschwungenen Verlauf entlang der Kreise K1 bzw. K2, wobei der Kreis K1 einen geringeren Radius aufweist als der Kreis K2. Die Kreise K1 und K2 verlaufen parallel zu einander und die Tangenten haben an jeder Position zueinander den Abstand K. Wäre der Abstand zwischen den Führungselementen 29 und Zapfen 25 gleich dem Abstand K, so könnte das Schiebeelement 10 entlang der Führungsnut 20 verschoben werden, ohne dass eine Schwenkbewegung des Trägers 28 um die Schwenkachse D entsteht, da auch die Führungselemente 29 ohne Widerstand der Führungskante 30 folgen könnten.

### Bezugszeichenliste

- 1: Maske
- 2: Maskenkörper
- 3: Gesichtspolster
- 4: Schlauchanschluss
- 5: Stirnstütze
- 6: Stützarm
- 7: Kopfbänderung
- 8: Bänderclip
- 9: Polsterhalter
- 10: Schiebeelement
- 11: Stirnpolster
- 12: Polsteranschluss
- 13: Cliphalter
- 14: Anschluss
- 15: Verschluss
- 16: Verbindungselement
- 17: Schiene
- 18: Gasanschluss
- 19: Halterrahmen
- 20: Führungsnut
- 21: Verschlusslasche
- 22: Verbindung
- 23: Verbindung
- 24: Griffrippe
- 25: Zapfen
- 26: Schieberplatte
- 27: Pin
- 28: Träger
- 29: Führungselement
- 30: Führungskante
- 31: Flügelelement
- 32: Innenwand
- 33: Einkerbung
- 34: Rastnase
- 35: Zwischenraum
- 36: Spalt
- 37: Freiraum
- 38: Polsteraufnahme
- 39: Stift
- 40: Tasche
- 41: Bänderaufnahme
- 42: Lücke
- 43: Zwischenraum
- 44: Unterflügel
- 45: Anschlussverriegelung
- 46: Wand
- 47: Haltermitte
- 48: Freiraum
- 49: Kante
- 50: Führungsnut
- 51: Oberkante
- A: Ebene
- B: Ebene
- C: Winkel
- D: Schwenkachse
- E: Außenseite
- F: Innenseite
- G: Symmetrieebene
- H: Abstand
- J: Abstand
- K: Abstand
- M: Abstand
- R1: Radius (Führungsnut 20)
- R2: Radius (Führungskante 30)
- R3: Radius (Führungselemente 29)
- K1: Kreis (Führungsnut 20)
- K2: Kreis (Führungskante 30)

## Patentansprüche

1. Stirnstützverstellung für eine Maske (1), wobei die Stirnstützverstellung zumindest umfasst
a. eine Stirnstütze (5), bestehend aus Stützarm (6) und Polsterhalter (9)
b. ein Verbindungselement (16)
c. eine Schiene (17) mit zumindest einer Führungsnut (20)
d. ein Schiebeelement (10)
wobei das Schiebeelement (10) zumindest eine Schieberplatte (26), zumindest einen Zapfen (25) sowie zumindest ein Führungselement (29) aufweist, wobei der zumindest eine Zapfen (25) in der zumindest einen Führungsnut (20) der Schiene (17) dreh- und schiebbar gelagert ist und wobei das Schiebeelement (10) mit dem Stützarm (6) beweglich verbunden ist,
wobei die Stirnstützverstellung so eingerichtet ist, dass ein Verschieben des Schiebeelements (10) entlang der Schiene (17) zu einem Schwenken der Stirnstütze (5) um mindestens eine Schwenkachse D führt,
wobei der Stützarm (6) zumindest zwei Träger (28) aufweist, wobei die Träger (28) parallel zueinander angeordnet sind und über Verbindungen (22, 23) miteinander verbunden sind, wobei die Verbindungen (22, 23) zusammen mit den Trägern (28) einen Freiraum (37) bilden, durch welchen das Schiebeelement (10) zumindest teilweise gesteckt ist,
wobei die Träger (28) zumindest eine Führungskante (30, 49) aufweisen, an welcher das zumindest eine Führungselement (29) entlanggeführt wird,
wobei zumindest einer der Träger (28) über zumindest einen Pin (27) mit dem Verbindungselement (16) drehbar verbunden ist.

2. Stirnstützverstellung nach Anspruch 1, **dadurch gekennzeichnet, dass** zumindest eine Innenwand (32) an der Schieberplatte (26) angeordnet ist, wobei an der Innenwand (32) der zumindest eine Zapfen (25) und das zumindest eine Führungselement (29) angeordnet sind.

3. Stirnstützverstellung nach zumindest einem der vorhergehenden Ansprüche, **dadurch gekennzeichnet, dass** in der Schiene (17) zumindest zwei Einkerbungen (33) angeordnet sind und an zumindest einem Zapfen (25) eine Rastnase (34) angeordnet ist, welche in den Einkerbungen (33) einrasten und dadurch eine Schwenkstellung fixieren kann

4. Stirnstützverstellung nach zumindest einem der vorhergehenden Ansprüche, **dadurch gekennzeichnet, dass** die Schieberplatte (26) an zwei sich gegenüberliegenden Seiten jeweils einen Flügel (31) aufweist, welche im Wesentlichen senkrecht zur Schieberplatte (26) stehen und die Flügel (31) zumindest jeweils zwei Unterflügel (44) aufweisen, wobei zwischen den jeweiligen Unterflügeln (44) ein Zwischenraum (43) vorhanden ist und wobei auf zumindest einer Seite der zumindest zwei Träger (28) diskrete Bezeichnungen zur Markierung der Schwenkstellung angeordnet sind, wobei die diskreten Bezeichnungen so angeordnet sind, dass zumindest eine Bezeichnung im Zwischenraum (43) zwischen den Unterflügeln (44) zu sehen ist, wenn die Rastnase (34) die entsprechende Schwenkstellung fixiert.

5. Stirnstützverstellung nach zumindest einem der vorhergehenden Ansprüche, **dadurch gekennzeichnet, dass** die Schiene (17) und das Verbindungselement (16) in den Maskenkörper (2) integrierbar sind.

6. Stirnstützverstellung nach zumindest einem der vorhergehenden Ansprüche, **dadurch gekennzeichnet, dass** an der Schieberplatte (26) zumindest zwei Innenwände (32) angeordnet sind, welche im Wesentlichen senkrecht an der Schieberplatte (26) angeordnet sind, und wobei an den Kanten, welche der Schieberplatte (26) gegenüberliegen, Zapfen (25) und Führungselemente (29) angeordnet sind

7. Stirnstützverstellung nach Anspruch 6, **dadurch gekennzeichnet, dass** die Führungselemente (29) und Zapfen (25) in einem Abstand J zueinander angeordnet sind und die zumindest eine Führungskante (30) und eine Oberkante (51) der zumindest einen Führungsnut (20) einen Abstand K zueinander aufweisen, wenn die zumindest eine Führungskante (30) und die zumindest eine Führungsnut (20) parallel zueinander verlaufen, wobei der Abstand J nicht gleich dem Abstand K ist und sich der Abstand J beim Bewegen des Schiebeelements (10) nicht verändert.

8. Stirnstützverstellung nach zumindest einem der vorhergehenden Ansprüche, **dadurch gekennzeichnet, dass** die zumindest eine Führungsnut (20) geschwungen oder gerade ausgeführt ist, wobei eine geschwungene Führungsnut (20) einen Radius R1 aufweist.

9. Stirnstützverstellung nach Anspruch 8, **dadurch gekennzeichnet, dass** die zumindest eine Führungskante (30, 49) geschwungen oder gerade ausgeführt ist, wobei eine geschwungene Führungskante (30, 49) einen Radius R2 aufweist, wobei der Radius R1 nicht gleich dem Radius R2 ist und das Verhältnis R2:R1 oder R1:R2 in einem Bereich von 0,5 bis 0,99, bevorzugt 0,7 bis 0,99, mehr bevorzugt 0,75 bis 0,985 liegt.

10. Stirnstützverstellung nach Anspruch 9, **dadurch gekennzeichnet, dass** die Radien R1 und R2 in einem Bereich zwischen 7 cm und 15 cm liegen, bevorzugt zwischen 9 cm und 11 cm

11. Stirnstützverstellung nach zumindest einem der vorhergehenden Ansprüche, **dadurch gekennzeichnet, dass** die Stirnstütze (5) um einen maximalen Schwenkwinkel C geschwenkt werden kann, wobei der maximale Schwenkwinkel C zwischen 10° und 40° liegt, bevorzugt zwischen 15° und 25°.

12. Stirnstützverstellung nach Anspruch 9 oder 10, **dadurch gekennzeichnet, dass** die Oberkante (51) der zumindest einen Führungsnut (20) auf einem Kreis K1 mit dem Radius R1 liegt und die zumindest eine Führungskante (30) auf einem Kreis K2 mit dem Radius R2 liegt.

13. Stirnstützverstellung nach Anspruch 12, **dadurch gekennzeichnet, dass** die Schwenkachse D immer die gleiche relative Position, also innerhalb oder außerhalb liegend, zu den Kreisen K1 und K2 hat, wenn der maximale Schwenkwinkel C nicht überschritten wird

14. Stirnstützverstellung nach Anspruch 12 oder 13, **dadurch gekennzeichnet, dass** die Schwenkachse D immer außerhalb des Kreises K2 liegt und immer innerhalb der Kreises K1.

15. Stirnstützverstellung nach zumindest einem der vorhergehenden Ansprüche, **dadurch gekennzeichnet, dass** ein Schieben des Schiebeelements (10) entlang der Schiene (17) in Richtung Polsterhalter (9) ein Annähern des Stützarms (6) an den Maskenkörper (2) bewirkt.

16. Stirnstützverstellung nach zumindest einem der vorhergehenden Ansprüche, **dadurch gekennzeichnet, dass** der Stützarm (6) an einem Ende in den Polsterhalter (9) übergeht, wobei an dem Ende des Stützarms (6), welches dem Ende mit dem Polsterhalter (9) gegenüberliegt, an der Verbindung (23) ein Stift (39) angeordnet ist, über welchen ein Verschluss (15) zum Verschließen eines Gasanschlusses (18) der Maske (1) unverlierbar mit der Maske (1) verbindbar ist, und der Polsterhalter (9) einen Halterrahmen (19) aufweist, welcher zusammen mit einer Haltermitte (47) einen Freiraum (48) bildet, in welchen zumindest eine Polsteraufnahme (38) von zumindest einer Seite des Halterrahmens (19) in Richtung Haltermitte (47) hineinragt und zur Aufnahme eines Stirnpolsters (11) dient.

17. Stirnstützverstellung nach Anspruch 6 oder Anspruch 7, **dadurch gekennzeichnet, dass** die Zapfen (25) und Führungselemente (29) so angeordnet und ausgebildet sind, dass ein Schieben des Schiebeelements (10) zu einem Verschieben der Zapfen (25) entlang der zumindest einen Führungsnut (20) und ein Verschieben der Führungselemente (29) entlang der zumindest einen Führungskante (30) erfolgt, wobei gleichzeitig die Stirnstütze (5) um die Schwenkachse D schwenkt und das Schiebeelement (10) um die Zapfen (25) gedreht wird.

18. Maske (1) mit einer Stirnstützverstellung nach zumindest einem der vorhergehenden Ansprüche, **dadurch gekennzeichnet, dass** zwischen einem Anschluss (14) und dem Verbindungselement (16) ein Gasanschluss (18) angeordnet ist.

19. Maske (1) nach Anspruch 18, **dadurch gekennzeichnet, dass** der Gasanschluss (18) ein Anschluss für eine zusätzliche Sauerstoffversorgung ist und der Gasanschluss (18) mit einem Verschluss (15) verschließbar ist, wobei dieser Verschluss (15) unverlierbar mit der Maske (1) über einen Stift (39) an der Stirnstütze (5) verbunden ist.

## Claims

1. A forehead support adjuster for a mask (1), wherein the forehead support adjuster comprises at least
a. a forehead support (5), consisting of a support arm (6) and a cushion holder (9)
b. a connecting element (16)
c. a rail (17) with at least one guide groove (20)
d. a sliding element (10)
wherein the sliding element (10) has at least one slider plate (26), at least one peg (25), and at least one guide element (29), wherein the at least one peg (25) is mounted so as to be rotatable and slidable in the at least one guide groove (20) of the rail (17) and
wherein the sliding element (10) is movably connected to the support arm (6),
wherein the forehead support adjuster is configured such that displacing the sliding element (10) along the rail (17) leads to a pivoting of the forehead support (5) about at least one pivot axis D,
wherein the support arm (6) has at least two carriers (28), wherein the carriers (28) are arranged parallel to each other and are connected to each other by means of connections (22, 23), wherein the connections (22, 23), together with the carriers (28), form a free space (37), through which the sliding element (10) is at least partially placed,
wherein the carriers (28) have at least one guide edge (30, 49), along which the at least one guide element (29) is guided,
wherein at least one of the carriers (28) is rotatably connected to the connecting element (16) by at least one pin (27).

2. The forehead support adjuster according to claim 1, **characterized in that** at least one inner wall (32) is arranged on the slider plate (26), wherein the at least one peg (25) and the at least one guide element (29) are arranged on the inner wall (32).

3. The forehead support adjuster according to at least one of the preceding claims, **characterized in that** at least two notches (33) are arranged in the rail (17) and a latching nose (34) is arranged on at least one peg (25) and can latch in the notches (33) and thereby fix a pivot position.

4. The forehead support adjuster according to at least one of the preceding claims, **characterized in that** the slider plate (26) has one wing (31) on each of two opposite sides, which wings are substantially perpendicular to the slider plate (26), and the wings (31) each have at least two lower wings (44), wherein a gap (43) is present between the respective lower wings (44) and wherein discrete designations for marking the pivot position are arranged on at least one side of the at least two carriers (28), wherein the discrete designations are arranged such that at least one designation can be seen in the gap (43) between the lower wings (44) when the latching nose (34) fixes the corresponding pivot position.

5. The forehead support adjuster according to at least one of the preceding claims, **characterized in that** the rail (17) and the connecting element (16) can be integrated into the mask body (2).

6. The forehead support adjuster according to at least one of the preceding claims, **characterized in that** at least two inner walls (32) are arranged on the slider plate (26) and are arranged substantially vertically on the slider plate (26), and wherein pegs (25) and guide elements (29) are arranged on the edges opposite the slider plate (26).

7. The forehead support adjuster according to claim 6, **characterized in that** the guide elements (29) and pegs (25) are arranged at a distance J from each other and the at least one guide edge (30) and an upper edge (51) of the at least one guide groove (20) have a distance K from each other when the at least one guide edge (30) and the at least one guide groove (20) run parallel to each other, wherein the distance J is not equal to the distance K and the distance J does not change when the sliding element (10) moves.

8. The forehead support adjuster according to at least one of the preceding claims, **characterized in that** the at least one guide groove (20) is curved or straight, wherein a curved guide groove (20) has a radius R1.

9. The forehead support adjuster according to claim 8, **characterized in that** the at least one guide edge (30, 49) is curved or straight, wherein a curved guide edge (30, 49) has a radius R2, wherein the radius R1 is not equal to the radius R2 and the ratio of R2:R1 or R1:R2 is in a range from 0.5 to 0.99, preferably 0.7 to 0.99, more preferably 0.75 to 0.985.

10. The forehead support adjuster according to claim 9, **characterized in that** the radii R1 and R2 are in a range between 7 cm and 15 cm, preferably between 9 cm and 11 cm.

11. The forehead support adjuster according to at least one of the preceding claims, **characterized in that** the forehead support (5) can be pivoted about a maximum pivot angle C, wherein the maximum pivot angle C is between 10° and 40°, preferably between 15° and 25°.

12. The forehead support adjuster according to claim 9 or 10, **characterized in that** the upper edge (51) of the at least one guide groove (20) is on a circle K1 with the radius R1 and the at least one guide edge (30) is on a circle K2 with the radius R2.

13. The forehead support adjuster according to claim 12, **characterized in that** the pivot axis D always has the same relative position in relation to the circles K1 and K2, meaning inside or outside of them, when the maximum pivot angle C is not exceeded.

14. The forehead support adjuster according to claim 12 or 13, **characterized in that** the pivot axis D is always outside the circle K2 and always inside the circle K1.

15. The forehead support adjuster according to at least one of the preceding claims, **characterized in that** sliding the sliding element (10) along the rail (17) in the direction of the cushion holder (9) causes the support arm (6) to approach the mask body (2).

16. The forehead support adjuster according to at least one of the preceding claims, **characterized in that** the support arm (6) transitions at one end into the cushion holder (9), wherein, at the end of the support arm (6) opposite the end with the cushion holder (9), at the connection (23), a stud (39) is arranged, by means of which a closure (15) for closing a gas connection (18) of the mask (1) can be captively connected to the mask (1), and the cushion holder (9) has a holder frame (19), which, together with a holder center (47), forms a free space (48), into which at least one cushion receiver (38) projects from at least one side of the holder frame (19) in the direction of the holder center (47) and serves to receive a forehead cushion (11).

17. The forehead support adjuster according to claim 6 or claim 7, **characterized in that** the pegs (25) and guide elements (29) are arranged and designed such that sliding the sliding element (10) to sliding of the pegs (25) along the at least one guide groove (20) and sliding of the guide elements (29) along the at least one guide edge (30) takes place, wherein at the same time the forehead support (5) pivots about the pivot axis D and the sliding element (10) is rotated about the pegs (25).

18. A mask (1) with a forehead support adjuster according to at least one of the preceding claims, **characterized in that** a gas connection (18) is arranged between a connection (14) and the connecting element (16).

19. The mask (1) according to claim 18, **characterized in that** the gas connection (18) is a connection for an additional oxygen supply and the gas connection (18) can be closed with a closure (15), wherein this closure (15) is captively connected to the mask (1) by means of a stud (39) on the forehead support (5).

## Revendications

1. Réglage de support frontal pour un masque (1), dans lequel le réglage de support frontal comporte au moins
a. un support frontal (5), constitué d'un bras de support (6) et d'un élément de maintien de coussinet (9)
b. un élément de liaison (16)
c. un rail (17) doté d'au moins une rainure de guidage (20)
d. un élément coulissant (10)
dans lequel l'élément coulissant (10) présente au moins une plaque de coulisseau (26), au moins un tenon (25) et au moins un élément de guidage (29), dans lequel l'au moins un tenon (25) est monté de façon rotative et coulissante dans l'au moins une rainure de guidage (20) du rail (17) et dans lequel l'élément coulissant (10) est relié de façon déplaçable au bras de support (6),
dans lequel le réglage de support frontal est configuré de telle façon qu'un coulissement de l'élément coulissant (10) le long du rail (17) entraîne un pivotement du support frontal (5) autour d'au moins un axe de pivotement D,
dans lequel le bras de support (6) présente au moins deux poutres (28), dans lequel les poutres (28) sont disposées parallèlement l'une à l'autre et reliées entre elles par des liaisons (22, 23), dans lequel les liaisons (22, 23) forment un espace libre (37) conjointement avec les poutres (28), à travers lequel l'élément coulissant (10) est au moins partiellement inséré,
dans lequel les poutres (28) présentent au moins un bord de guidage (30, 49) le long duquel l'au moins un élément de guidage (29) est guidé,
dans lequel l'une au moins des poutres (28) est reliée de façon rotative à l'élément de liaison (16) par le biais d'au moins une broche (27).

2. Réglage de support frontal selon la revendication 1, **caractérisé en ce qu'**au moins une paroi intérieure (32) est disposée sur la plaque de coulisseau (26), dans lequel l'au moins un tenon (25) et l'au moins un élément de guidage (29) sont disposés sur la paroi intérieure (32).

3. Réglage de support frontal selon l'une au moins des revendications précédentes, **caractérisé en ce qu'**au moins deux encoches (33) sont disposées dans le rail (17) et un nez d'encliquetage (34) est disposé sur l'au moins un tenon (25), lequel peut s'encliqueter dans les encoches (33) et ainsi fixer une position de pivotement.

4. Réglage de support frontal selon l'une au moins des revendications précédentes, **caractérisé en ce que** la plaque de coulisseau (26) présente respectivement une aile (31) sur deux côtés opposés, lesquelles sont essentiellement perpendiculaires à la plaque de coulisseau (26) et les ailes (31) présentent au moins respectivement deux ailes inférieures (44), dans lequel un espace intermédiaire (43) est présent entre les ailes inférieures (44) respectives et dans lequel des étiquettes discrètes sont disposées sur au moins un côté des au moins deux poutres (28) pour le marquage de la position de pivotement, dans lequel les étiquettes discrètes sont disposées de telle façon qu'au moins une étiquette est visible dans l'espace intermédiaire (43) entre les ailes inférieures (44) lorsque le nez d'encliquetage (34) fixe la position de pivotement correspondante.

5. Réglage de support frontal selon l'une au moins des revendications précédentes, **caractérisé en ce que** le rail (17) et l'élément de liaison (16) peuvent être intégrés dans le corps de masque (2).

6. Réglage de support frontal selon l'une au moins des revendications précédentes, **caractérisé en ce qu'**au moins deux parois intérieures (32) sont disposées sur la plaque de coulisseau (26), lesquelles sont disposées essentiellement perpendiculairement sur la plaque de coulisseau (26), et dans lequel des tenons (25) et des éléments de guidage (29) sont disposés sur les bords opposés à la plaque de coulisseau (26).

7. Réglage de support frontal selon la revendication 6, **caractérisé en ce que** les éléments de guidage (29) et les tenons (25) sont disposés à une distance J les uns des autres et l'au moins un bord de guidage (30) et un bord supérieur (51) de l'au moins une rainure de guidage (20) présentent une distance K entre eux lorsque l'au moins un bord de guidage (30) et l'au moins une rainure de guidage (20) s'étendent parallèlement l'un à l'autre, dans lequel la distance J est différente de la distance K et la distance J ne change pas lors du déplacement de l'élément coulissant (10).

8. Réglage de support frontal selon l'une au moins des revendications précédentes, **caractérisé en ce que** l'au moins une rainure de guidage (20) est conçue incurvée ou rectiligne, dans lequel une rainure de guidage incurvée (20) présente un rayon R1.

9. Réglage de support frontal selon la revendication 8, **caractérisé en ce que** l'au moins un bord de guidage (30, 49) est conçu incurvé ou rectiligne, dans lequel un bord de guidage incurvé (30, 49) présente un rayon R2, dans lequel le rayon R1 est différent du rayon R2 et le rapport R2:R1 ou R1:R2 est compris dans la plage de 0,5 à 0,99, préférentiellement de 0,7 à 0,99, plus préférentiellement de 0,75 à 0,985.

10. Réglage de support frontal selon la revendication 9, **caractérisé en ce que** les rayons R1 et R2 sont compris dans une plage entre 7 cm et 15 cm, préférentiellement entre 9 cm et 11 cm.

11. Réglage de support frontal selon l'une au moins des revendications précédentes, **caractérisé en ce que** le support frontal (5) peut pivoter selon un angle de pivotement maximal C, dans lequel l'angle de pivotement maximal C est compris entre 10° et 40°, préférentiellement entre 15° et 25°.

12. Réglage de support frontal selon la revendication 9 ou 10, **caractérisé en ce que** le bord supérieur (51) de l'au moins une rainure de guidage (20) se trouve sur un cercle K1 avec le rayon R1 et l'au moins un bord de guidage (30) se trouve sur un cercle K2 avec le rayon R2.

13. Réglage de support frontal selon la revendication 12, **caractérisé en ce que** l'axe de pivotement D a toujours la même position relative, donc située à l'intérieur ou à l'extérieur, par rapport aux cercles K1 et K2, lorsque l'angle de pivotement maximal C n'est pas dépassé.

14. Réglage de support frontal selon la revendication 12 ou 13, **caractérisé en ce que** l'axe de pivotement D se trouve toujours à l'extérieur du cercle K2 et toujours à l'intérieur du cercle K1.

15. Réglage de support frontal selon l'une au moins des revendications précédentes, **caractérisé en ce qu'**un coulissement de l'élément coulissant (10) le long du rail (17) en direction de l'élément de maintien de coussinet (9) entraîne un rapprochement du bras de support (6) vers le corps de masque (2).

16. Réglage de support frontal selon l'une au moins des revendications précédentes, **caractérisé en ce que** le bras de support (6) se prolonge dans l'élément de maintien de coussinet (9) à une extrémité, dans lequel, à l'extrémité du bras de support (6) opposée à l'extrémité comprenant l'élément de maintien de coussinet (9), une tige (39) est disposée au niveau de la liaison (23), par le biais de laquelle un moyen de fermeture (15) destiné à fermer un raccord de gaz (18) du masque (1) peut être relié de façon imperdable au masque (1), et l'élément de maintien de coussinet (9) présente un cadre d'élément de maintien (19), lequel forme un espace libre (48) conjointement avec un centre d'élément de maintien (47), dans lequel au moins un élément de réception de coussinet (38) fait saillie depuis au moins un côté du cadre d'élément de maintien (19) en direction du centre d'élément de maintien (47) et sert à recevoir un coussinet frontal (11).

17. Réglage de support frontal selon la revendication 6 ou la revendication 7, **caractérisé en ce que** les tenons (25) et les éléments de guidage (29) sont disposés et réalisés de telle façon qu'une poussée de l'élément coulissant (10) amène un coulissement des tenons (25) le long de l'au moins une rainure de guidage (20) et un coulissement des éléments de guidage (29) le long de l'au moins un bord de guidage (30), dans lequel le support frontal (5) pivote autour de l'axe de pivotement D et l'élément coulissant (10) tourne simultanément autour des tenons (25).

18. Masque (1) doté d'un réglage de support frontal selon l'une au moins des revendications précédentes, **caractérisé en ce qu'**un raccord de gaz (18) est disposé entre un raccord (14) et l'élément de liaison (16).

19. Masque (1) selon la revendication 18, **caractérisé en ce que** le raccord de gaz (18) est un raccord pour une alimentation en oxygène supplémentaire et le raccord de gaz (18) peut être fermé à l'aide d'un élément de fermeture (15), dans lequel cet élément de fermeture (15) est relié de façon imperdable au masque (1) par le biais d'une tige (39) sur le support frontal (5).
